# EUROPÄISCHE PATENTSCHRIFT

(11) **EP 3 880 650 B1**
(45) Veröffentlichungstag und Bekanntmachung des Hinweises auf die Patenterteilung: **06.09.2023**
(21) Anmeldenummer: 19702041.5
(22) Anmeldetag: 23.01.2019
(51) Int. Cl.: C07C 69/675, A61P 1/00, A61K 31/22, A61P 3/00, C07C 69/716

(54) **VERFAHREN ZUR HERSTELLUNG VON STRUKTUREINHEITEN AUF BASIS VON GLYCERIDEN VON HYDROXYCARBONSÄUREN ENTHALTENDEN LIPIDEN**
METHOD FOR PRODUCING LIPIDS CONTAINING STRUCTURAL UNITS ON THE BASIS OF GLYCERIDES OF HYDROXY CARBOXYLIC ACIDS
PROCÉDÉ DE PRÉPARATION DE LIPIDES CONTENANT DES MOTIFS STRUCTURAUX À BASE DE GLYCÉRIDES D'ACIDES HYDROXYCARBOXYLIQUES

(30) Priorität: 17.01.2019 WO PCT/EP2019/051120
(43) Veröffentlichungstag der Anmeldung: 22.09.2021
(73) Patentinhaber: KetoLipix Therapeutics GmbH, 20459 Hamburg (DE)
(72) Erfinder: LOCHMANN, Dirk, 58453 Witten (DE); REYER, Sebastian, 58453 Witten (DE); STEHR, Michael, 58453 Witten (DE)
(74) Vertreter: Strehlke, Ingo Kurt
(86) Internationale Anmeldenummer: PCT/EP2019/051541
(87) Internationale Veröffentlichungsnummer: WO 2020/147980

(56) Entgegenhaltungen:
- WO-A1-2013/150153
- WO-A1-2017/147220
- US-A- 5 693 850
- US-A1- 2018 303 821

## Beschreibung

Die vorliegende Erfindung betrifft das Gebiet der Ketokörper und des damit zusammenhängenden Stoffwechsels sowie die Therapie von damit im Zusammenhang stehenden Erkrankungen.

Insbesondere betrifft die vorliegende Erfindung ein Verfahren zur Herstellung von Lipiden, welche Struktureinheiten auf Basis von Glyceriden von 3-Hydroxy- und/oder 3-Alkoxybuttersäuren enthalten, sowie die auf diese Weise erhältlichen bzw. hergestellten Reaktionsprodukte (d. h. Struktureinheiten auf Basis von Glyceriden von 3-Hydroxy- und/oder 3-Alkoxybuttersäuren enthaltende Lipide) und deren Verwendung, insbesondere in pharmazeutischen Zusammensetzungen, wie Arzneimitteln oder Medikamenten, oder in Nahrungsmittel- und/oder Lebensmittelerzeugnissen, sowie deren weitere Anwendungen bzw. Verwendungen.

Des Weiteren betrifft die vorliegende Erfindung pharmazeutische Zusammensetzungen, insbesondere Arzneimittel oder Medikamente, welche die nach dem erfindungsgemäßen Herstellungsverfahren erhältlichen bzw. hergestellten Reaktionsprodukte (d. h. Struktureinheiten auf Basis von Glyceriden von 3-Hydroxy-und/oder 3-Alkoxybuttersäuren enthaltende Lipide) umfassen, sowie deren Anwendungen bzw. Verwendungen.

Weiterhin betrifft die vorliegende Erfindung Nahrungsmittel- und/oder Lebensmittelerzeugnisse, insbesondere Nahrungsergänzungsmittel, funktionelle Lebensmittel (*Functional Food*)*, Novel Food,* Lebensmittelzusatzstoffe, Nahrungszusätze, diätetische Lebensmittel, Power-Snacks, Appetitzügler und Kraft-und/oder Ausdauersport-Supplements, welche die nach dem erfindungsgemäßen Herstellungsverfahren erhältlichen bzw. hergestellten Reaktionsprodukte (d. h. Struktureinheiten auf Basis von Glyceriden von 3-Hydroxy- und/oder 3-Alkoxybuttersäuren enthaltende Lipide) umfassen, sowie deren Anwendungen bzw. Verwendungen.

Im menschlichen Energiestoffwechsel ist Glucose der kurzfristig zur Verfügung stehende Energieträger, welcher in den Mitochondrien unter Freisetzung von Wasser und Kohlendioxid zu Energie verstoffwechselt wird. Bereits durch die Schlafperiode während der Nacht sind aber die Glycogenspeicher der Leber geleert. Jedoch benötigen vor allem das menschliche zentrale Nervensystem (ZNS) und das Herz eine permanente Energieversorgung.

Die physiologische Alternative zu Glucose, welche vor allem dem zentralen Nervensystem zur Verfügung steht, sind die sogenannten Ketokörper (synonym auch als Ketonkörper bezeichnet oder Englisch auch als *"Keton Bodies"* bezeichnet).

Der Begriff der Ketokörper ist insbesondere eine Sammelbezeichnung für drei Verbindungen, welche vor allem in katabolen Stoffwechsellagen (wie z. B. bei Hunger, Reduktionsdiät oder kohlenhydratarmer Ernährung) gebildet werden und unter Umständen zu einer Ketose führen. Unter den Begriff der Ketokörper fasst man insbesondere die drei Verbindungen Acetoacetat (synonym auch Acetacetat genannt) und Aceton sowie 3-Hydroxybuttersäure (nachfolgend synonym auch als beta-Hydroxybuttersäure oder BHB oder 3-BHB bezeichnet) bzw. deren Salz (d. h. 3-Hydroxybutyrat oder beta-Hydroxybutyrat) zusammen, wobei letztere Verbindung die bedeutendste der drei vorgenannten Verbindungen ist. 3-Hydroxybuttersäure bzw. deren Salz kommt physiologisch als (R)-Enantiomer vor, d. h. als (R)-3-Hydroxybuttersäure (synonym auch (3R)-3-Hydroxybuttersäure genannt, um das Chiralitätszentrum in 3-Position hervorzuheben) bzw. deren Salz.

Diese Ketokörper werden auch in großer Zahl beim Fasten oder Hungern physiologisch aus im Körper eingelagerten Lipiden durch Lipolyse bereitgestellt und ersetzen den Energieträger Glucose fast vollständig.

Die Ketokörper werden in der Leber aus Acetyl-Coenzym A (= Acetyl-CoA) gebildet, welches aus der beta-Oxidation stammt; sie stellen eine transportable Form des Acetyl-Coenzyms A im menschliche Körper dar. Zur Verwertung der Ketokörper müssen sich Gehirn und Muskeln aber zunächst umstellen, indem sie Enzyme exprimieren, welche zur Rückwandlung von Ketokörpern in Acetyl-Coenzym A benötigt werden. Insbesondere in Hungerzeiten tragen die Ketokörper einen beträchtlichen Anteil zur Energiegewinnung bei. So ist es beispielsweise dem Gehirn nach einiger Zeit möglich, mit nur einem Drittel der Tagesmenge an Glucose auszukommen.

Physiologisch erfolgt die Synthese der Ketokörper aus zwei Molekülen aktivierter Essigsäure in Form von Acetyl-Coenzym A, dem normalen Zwischenprodukt des Fettsäureabbaus, wobei zunächst mit Hilfe der Acetyl-Coenzym A-Acetyltransferase das Acetoacetyl-Coenzym A gebildet wird, welches unter Verwendung einer weiteren Acetyl-Coenzym A-Einheit und des Enzyms HMG-CoA-Synthase zum Zwischenprodukt 3-Hydroxy-3-methyl-glutaryl-CoA (HMG-CoA) verlängert wird, wobei schließlich die HMG-CoA-Lyase das Acetoacetat abspaltet. Diese drei Schritte finden ausschließlich in den Mitrochondrien der Leber statt (Lynenzyklus), wobei 3-Hydroxybutyrat schließlich im Zytosol durch die D-beta-Hydroxybutyrat-Dehydrogenase entsteht. HMG-CoA ist außerdem ein Endprodukt beim Abbau der Aminosäure Leucin, während Acetoacetat beim Abbau der Aminosäuren Phenylalanin und Tyrosin entsteht.

Durch spontane Decarboxylierung entsteht aus Acetoacetat Aceton; es ist gelegentlich im Atem von Diabetikern und Diäthaltenden wahrzunehmen. Es kann vom Körper nicht weiterverwendet werden. Der Anteil von Aceton an den Ketokörpern ist allerdings gering.

Acetoacetat wird also reduktiv in die physiologisch relevante Form der 3-Hydroxybuttersäure bzw. des 3-Hydroxybutyrats überführt, kann aber auch unter Kohlenstoffdioxidfreisetzung in das physiologisch unbrauchbare Aceton zerfallen, was bei einer schweren Ketose, einer Ketoazidose (z. B. bei Diabetes mellitus Typ 1-Patienten ohne Insulinsubstitution), im Urin und in der Ausatemluft nachweisbar und olfaktorisch wahrnehmbar ist.

3-Hydroxybuttersäure wird derzeit im Bereich des Kraftsports als Natrium-, Magnesium- oder Calcium-Salz eingesetzt und in den Handel gebracht.

Jedoch ist 3-Hydroxybuttersäure für den Menschen evolutionär nicht oder in nur sehr geringer Menge bekannt, da Pflanzen keine 3-Hydroxybuttersäure produzieren und 3-Hydroxybuttersäure im tierischen Organismus nur bei toten ausgezehrten Tieren in der Ketose vorkommt, so dass 3-Hydroxybuttersäure bei peroraler Verabreichung Brechreiz auslöst. 3-Hydroxybuttersäure in Form der freien Säure sowie deren Salzen schmeckt zudem stark bitter und kann schweres Erbrechen und Übelkeit hervorrufen.

Zudem können Patienten, vor allem Neugeborene, aber auch Erwachsene größere Mengen an Salzen der 3-Hydroxybuttersäure nicht permanent verkraften, da diese Verbindungen nierenschädigend wirken können.

Außerdem ist die Plasmahalbwertszeit von 3-Hydroxybuttersäure und deren Salzen derart gering, dass selbst bei Einnahme von mehreren Gramm die Ketose nur für ca. drei bis vier Stunden vorhält, d. h. Patienten können insbesondere während der Nacht daher nicht von einer Therapie mit 3-Hydroxybuttersäure oder deren Salzen kontinuierlich profitieren. Bei Stoffwechselerkrankungen kann dies zu lebensbedrohlichen Situationen führen.

Daher werden im Fall der Therapie derartiger Stoffwechselerkrankungen heute sogenannte mittelkettige Triglyceride, sogenannte MCTs, für die ketogene Therapie eingesetzt, d. h. es wird die metabolische Umwandlung von Capron-, Capryl- und Caprinsäure (d. h. von gesättigten linearen C₆-, C₈- und C₁₀-Fettsäuren) aus den korrespondierenden Triglyceriden beabsichtigt.

Grundsätzlich stellt aber aus pharmazeutischer und klinischer Hinsicht 3-Hydroxybuttersäure demgegenüber ein wirksameres pharmazeutisch-pharmakologisches Zielmolekül, welches nach den Erkenntnissen des Standes der Technik prinzipiell für die Therapie einer Vielzahl von Erkrankungen zum Einsatz kommen könnte, aber aufgrund seiner mangelnden physiologischen Kompatibilität dort nicht zum Einsatz kommen kann (z. B. bei Erkrankungen im Zusammenhang mit einer Störung des Energiestoffwechsels, insbesondere Ketokörperstoffwechsels, oder neurodegenerativen Erkrankungen wie Demenz, Morbus Alzheimer, Morbus Parkinson etc., Fettstoffwechselerkrankungen usw.).

Die nachfolgende Tabelle veranschaulicht rein beispielhaft, aber keinesfalls beschränkend potentielle Therapiemöglichkeiten bzw. mögliche Indikationen für den Wirkstoff 3-Hydroxybuttersäure.

| **Indikation** | **Therapeutischer Effekt** |
|---|---|
| Schädel-Hirn-Trauma | Unter BHB sinkt die Apoptose und Nekrose-Rate von Nervenzellen. |
| Schlaganfall | Unter BHB sinkt die Apoptose und Nekrose-Rate von Nervenzellen. |
| Refeeding-Syndrom | Bei Anorexie, Absetzen enteraler oder parenteraler Ernährung und nach langen Hungerperioden kann der Konsum von Stärke oder Glucose zum Tod führen (siehe auch WHO-Schema Erdnusspaste). BHB kann hier als Therapeutikum zum schnelleren Erlangen einer normalen Nahrungsaufnahme eingesetzt werden. |
| Appetitzügler | BHB unterdrückt im Zentralnervensystem (ZNS) das Hungergefühl. |
| Epilepsie | Herkömmliche ketogene Diät zur signifikanten Reduzierung der Häufigkeit von Krampfanfällen hat extrem schlechte Patienten-Verträglichkeit. BHB bietet hier eine unmittelbar wirksame Alternative. |
| Morbus Alzheimer, Demenz | Unter BHB zeigen Patienten eine bessere kognitive Leistung. BHB ist auch für die Prävention von neurodegenerativen Erkrankungen wirksam. |
| Störungen der Fettsäureoxidation (z. B. Electron-Transfer-Protein Defekt) | Ausgleich eines Nährstoffmangels bei Defekt im Energiestoffwechsel. |

Daher ist es aus pharmazeutischer und klinischer Hinsicht wünschenswert, wirksame Präkursoren oder Metabolite auffinden zu können, welche physiologisch einen direkten oder indirekten Zugang zu 3-Hydroxybuttersäure oder deren Salzen ermöglichen, insbesondere im physiologischen Stoffwechsel des menschlichen oder tierischen Körpers.

Folglich hat es im Stand der Technik nicht an Versuchen gefehlt, physiologisch geeignete Präkursoren oder Metaboliten für 3-Hydroxybuttersäure bzw. deren Salze aufzufinden. Bislang wurden im Stand der Technik jedoch keine effizienten diesbezüglichen Verbindungen aufgefunden. Auch ist ein diesbezüglicher Zugang zu solchen Verbindungen nach dem Stand der Technik bislang nicht bzw. nicht ohne Weiteres möglich.

Die WO 2017/147220 A1 betrifft Kombinationstherapie-Technologien, welche zur Behandlung einer oder mehrerer Krankheiten, Störungen oder Zustände nützlich sein sollen, welche mit einem abnormalen Metabolismus zusammenhängen können, wobei Kombinationen von TCA-Zyklus-Säuren und Ketokörpern oder aber Kombinationen von TCA-Zyklus-Säuren und anderen Carbonsäuren verwendet werden.

Weiterhin betrifft die WO 2013/150153 Ketokörper und Ketokörperester zur oralen Verabreichung zu Zwecken der Verbesserung oder zum Erhalt der Muskelkraft, wobei bestimmte Ester von Hydroxybutyratmonomeren organoleptisch akzeptabel sein und zu einer hohen Aufnahme vom Darm ins Blut führen sollen, wodurch ein schneller Anstieg der Hydroxybutyratkonzentration im Blut und eine physiologische Reaktion einschließlich einer verbesserten Leistungsabgabe während des Trainings ermöglicht werden soll, sowie darüber hinaus Zusammensetzungen, welche die Ketokörper oder Ketokörperester enthalten.

Die US 5 693 850 A betrifft ein Verfahren zur Herstellung von wasserlöslichen Glycerinestern, welche als parenterale Nährstoffe nützlich sein sollen, wobei ein Glycerin oder ein geschütztes Glycerin und ein Acetoacetatester oder Acetoacetatvorläufer zur Reaktion gebracht werden, wobei ein Acetoacetylglycerin erhalten wird, welches anschließend reduziert wird, wobei ein Glycerinester von 3-Hydroxybuttersäure erhalten wird.

Schließlich betrifft die US 2018/303821 ein Verfahren zum Initiieren von Anästhesie, Sedierung und ein Verfahren zur Behandlung von Störungen, einschließlich Störungen des zentralen Nervensystems, Störungen des peripheren Nervensystems, Depressionschemie und Behandlung mit einem Antikonvulsivum durch Verabreichung einer wirksamen Menge von bestimmten, dort definierten chemischen Verbindungen an einem Patienten.

Das der vorliegenden Erfindung zugrundeliegende Problem liegt also in der Bereitstellung eines effizienten Herstellungsverfahrens von physiologisch geeigneten bzw. physiologisch kompatiblen Präkursoren und/oder Metaboliten von 3-Hydroxybuttersäure (d. h. synonym auch beta-Hydroxybuttersäure bzw. BHB bzw. 3-BHB genannt) oder deren Salzen.

Ein solches Verfahren soll insbesondere die betreffenden BHB-Präkursoren und/oder BHB-Metaboliten in effizienter Weise zugänglich machen, insbesondere auch in größeren Mengen und ohne nennenswerte Mengen an toxischen Nebenprodukten.

In vollkommen überraschender Weise hat die Anmelderin nunmehr herausgefunden, dass Lipide, welche Struktureinheiten auf Basis von Glyceriden von 3-Hydroxy-und/oder 3-Alkoxybuttersäuren enthalten, einen effizienten und physiologisch wirksamen bzw. physiologisch kompatiblen Präkursor und/oder Metaboliten für den Ketokörper 3-Hydroxybuttersäure bzw. deren Salze darstellen, und hat in diesem Zusammenhang ein effizientes Herstellungsverfahren für diese Verbindungen auffinden bzw. entwickeln können, welches einen direkten und wirksamen, insbesondere ökonomischen wie auch großtechnisch umsetzbaren Zugang zu diesen Verbindungen ermöglicht.

Zur Lösung des zuvor geschilderten Problems schlägt die vorliegende Erfindung daher - gemäß einem **ersten** Aspekt der vorliegenden Erfindung - ein Verfahren zur Herstellung von Struktureinheiten auf Basis von Glyceriden von 3-Hydroxy-und/oder 3-Alkoxybuttersäuren enthaltenden Lipiden gemäß Anspruch 1 vor; weitere, insbesondere besondere und/oder vorteilhafte Ausgestaltungen des erfindungsgemäßen Verfahrens sind Gegenstand der diesbezüglichen Verfahrensunteransprüche.

Weiterhin betrifft die vorliegende Erfindung - gemäß einem **zweiten** Aspekt der vorliegenden Erfindung - ein nach dem erfindungsgemäßen Verfahren erhältliches Gemisch von mindestens zwei, insbesondere mindestens drei, voneinander verschiedenen, Struktureinheiten auf Basis von Glyceriden von 3-Hydroxy- und/oder 3-Alkoxybuttersäuren enthaltende Lipiden gemäß dem diesbezüglichen Anspruch (Anspruch 5); weitere, insbesondere besondere und/oder vorteilhafte Ausgestaltungen dieses Erfindungsaspekts sind Gegenstand der diesbezüglichen Unteransprüche.

Gleichermaßen betrifft die vorliegende Erfindung - gemäß einem **dritten** Aspekt der vorliegenden Erfindung - eine pharmazeutische Zusammensetzung, insbesondere Arzneimittel oder Medikament, gemäß dem diesbezüglichen unabhängigen Anspruch (Anspruch 15); weitere, insbesondere besondere und/oder vorteilhafte Ausgestaltungen dieses Erfindungsaspekts sind Gegenstand des diesbezüglichen Unteranspruchs.

Darüber hinaus betrifft die vorliegende Erfindung - gemäß einem **vierten** Aspekt der vorliegenden Erfindung -ein erfindungsgemäßes Gemisch von mindestens zwei, insbesondere mindestens drei voneinander verschiedenen, Struktureinheiten auf Basis von Glyceriden von 3-Hydroxy- und/oder 3-Alkoxybuttersäuren enthaltenden Lipiden zur prophylaktischen und/oder therapeutischen Behandlung bzw. zur Verwendung bei der prophylaktischen und/oder therapeutischen Behandlung von Erkrankungen des menschlichen oder tierischen Körpers gemäß dem diesbezüglichen unabhängigen Anspruch (Anspruch 17).

Des Weiteren betrifft die vorliegende Erfindung - gemäß einem **fünften** Aspekt der vorliegenden Erfindung - die Verwendung eines erfindungsgemäßen Gemischs von mindestens zwei, insbesondere mindestens drei voneinander verschiedenen, Struktureinheiten auf Basis von Glyceriden von 3-Hydroxy- und/oder 3-Alkoxybuttersäuren enthaltenden Lipiden zur prophylaktischen und/oder therapeutischen Behandlung bzw. zur Herstellung eines Arzneimittels zur prophylaktischen und/oder therapeutischen Behandlung von Erkrankungen des menschlichen oder tierischen Körpers gemäß dem diesbezüglichen unabhängigen Anspruch (Anspruch 18).

Des Weiteren betrifft die vorliegende Erfindung - gemäß einem **sechsten** Aspekt der vorliegenden Erfindung - ein Nahrungsmittel und/oder Lebensmittelerzeugnis gemäß dem diesbezüglichen unabhängigen Anspruch (Anspruch 19); weitere, insbesondere besondere und/oder vorteilhafte Ausgestaltungen des erfindungsgemäßen Nahrungsmittel- und/oder Lebensmittelerzeugnisses sind Gegenstand des diesbezüglichen Unteranspruchs. Schließlich betrifft die vorliegende Erfindung - gemäß einem **siebten** Aspekt der vorliegenden Erfindung - die Verwendung eines erfindungsgemäßen Gemischs von mindestens zwei, insbesondere mindestens drei voneinander verschiedenen, Struktureinheiten auf Basis von Glyceriden von 3-Hydroxy- und/oder 3-Alkoxybuttersäuren enthaltenden Lipiden in einem Nahrungsmittel- und/oder Lebensmittelerzeugnis gemäß dem diesbezüglichen unabhängigen Anspruch (Anspruch 20).

Es versteht sich bei den nachfolgenden Ausführungen von selbst, dass Ausgestaltungen, Ausführungsformen, Vorteile und dergleichen, welche nachfolgend zu Zwecken der Vermeidung von Wiederholungen nur zu einem Erfindungsaspekt ausgeführt sind, selbstverständlich auch in Bezug auf die übrigen Erfindungsaspekte entsprechend gelten, ohne dass dies einer gesonderten Erwähnung bedarf.

Des Weiteren versteht es sich von selbst, dass einzelne Aspekte und Ausführungsformen der vorliegenden Erfindung auch in beliebiger Kombination mit anderen Aspekten und Ausführungsformen der vorliegenden Erfindung als offenbart gelten und insbesondere auch eine beliebige Kombination von Merkmalen und Ausführungsformen, wie sie sich aus den Rückbezügen aller Patentansprüche ergibt, umfangreich als offenbart gilt, und zwar im Hinblick auf alle sich ergebenden Kombinationsmöglichkeiten.

Bei allen nachstehend genannten relativen bzw. prozentualen gewichtsbezogenen Angaben, insbesondere relativen Mengen- oder Gewichtsangaben, ist weiterhin zu beachten, dass diese im Rahmen der vorliegenden Erfindung vom Fachmann derart auszuwählen sind, dass sie sich in der Summe unter Einbeziehung aller Komponenten bzw. Inhaltsstoffe, insbesondere wie nachfolgend definiert, stets zu 100 % bzw. 100 Gew.-% ergänzen bzw. addieren; dies versteht sich aber für den Fachmann von selbst.

Im Übrigen gilt, dass der Fachmann - anwendungsbezogen oder aber einzelfallbedingt - von den nachfolgend angeführten Bereichsangaben erforderlichenfalls abweichen kann, ohne dass er den Rahmen der vorliegenden Erfindung verlässt.

Zudem gilt, dass alle im Folgenden genannten Werte- bzw. Parameterangaben oder dergleichen grundsätzlich mit genormten bzw. standardisierten oder explizit angegebenen Bestimmungsverfahren oder andernfalls mit dem Fachmann auf diesem Gebiet an sich geläufigen Bestimmungs- bzw. Messmethoden ermittelt bzw. bestimmt werden können.

Dies vorausgeschickt, wird die vorliegende Erfindung nunmehr nachfolgend im Detail erläutert.

Gegenstand der vorliegenden Erfindung - gemäß einem **ersten** Aspekt der vorliegenden Erfindung - ist ein
Verfahren zur Herstellung von Lipiden, welche Struktureinheiten auf Basis von Glyceriden von 3-Hydroxy- und/oder 3-Alkoxybuttersäuren enthalten,
wobei mindestens ein Glycerid der allgemeinen Formel (I)

   R¹O-CH₂-CH(OR²)-CH₂-OR³ (I)

   wobei in der allgemeinen Formel (I) die Reste R¹, R² und R³, gleich oder verschieden, jeweils unabhängig voneinander
   - Wasserstoff,
   - einen Rest C₁-C₁₁-Alkyl-C(O)- , insbesondere C₂-C₁₁-Alkyl-C(O)-,
   - einen Rest C₁₂-C₂₉-Alkyl-C(O)- , insbesondere C₁₉-C₂₉-Alkyl-C(O)-, darstellen, jedoch mit der Maßgabe, dass mindestens einer, vorzugsweise mindestens zwei der Reste R¹, R² und R³ keinen Wasserstoff darstellen,
mit mindestens einem 3-Hydroxy- und/oder 3-Alkoxybuttersäure-Derivat der allgemeinen Formel (II)

   CH₃-CH(OR⁴)-CH₂-C(O)OR⁵ (II)

   wobei in der allgemeinen Formel (II)
   - der Rest R⁴ Wasserstoff oder einen Rest C₁-C₃₀-Alkyl-C(O)-, insbesondere C₁-C₂₁-Alkyl-C(O)- , vorzugsweise C₃-C₂₁-Alkyl-C(O)-, darstellt,
   - der Rest R⁵ Wasserstoff oder ein C₁-C₄-Alkyl, insbesondere ein C₁-C₄-Alkyl, bevorzugt Methyl oder Ethyl, besonders bevorzugt Ethyl, darstellt,
umgesetzt wird,
wobei die Umsetzung in Abwesenheit von Lösemitteln und/oder ohne jedwedes Lösemittel durchgeführt wird und
wobei die Umsetzung in Gegenwart eines Enzyms als Katalysator durchgeführt wird, so dass als Reaktionsprodukt ein Gemisch von mindestens zwei voneinander verschiedenen, Struktureinheiten auf Basis von Glyceriden von 3-Hydroxy- und/oder 3-Alkoxybuttersäuren enthaltenden Lipiden der allgemeinen Formel (III)

   R⁶O-CH₂-CH(OR⁷)-CH₂-OR⁸ (III)

   wobei in der allgemeinen Formel (III) die Reste R⁶, R⁷ und R⁸, gleich oder verschieden, jeweils unabhängig voneinander
      - einen Rest C₁-C₁₁-Alkyl-C(O)-, insbesondere C₂-C₁₁-Alkyl-C(O)-,
      - einen Rest C₁₂-C₂₉-Alkyl-C(O)-, insbesondere C₁₉-C₂₉-Alkyl-C(O)-,
      - einen Rest CH₃-CH(OR⁴)-CH₂-C(O)-, worin der Rest R⁴ die zuvor angegebene Bedeutung hat,
   darstellen, jedoch mit der Maßgabe, dass einer oder zwei der Reste R⁶, R⁷ und R⁸ einen Rest CH₃-CH(OR⁴)-CH₂-C(O)-darstellt,
erhalten wird.

Wie zuvor ausgeführt, hat die Anmelderin nämlich vollkommen überraschend herausgefunden, dass die auf diese Weise hergestellten Lipide, welche Struktureinheiten auf Basis von Glyceriden von 3-Hydroxy- und/oder 3-Alkoxybuttersäuren enthalten, effiziente, da physiologisch verträgliche Präkursoren und/oder Metabolite der freien 3-Hydroxybuttersäure bzw. deren Salzen darstellen, welche pharmazeutisch bzw. klinisch auch in größeren Mengen zum Einsatz kommen können, da sie physiologisch kompatibel sind.

Die vorgenannten Lipide, welche Struktureinheiten auf Basis von Glyceriden von 3-Hydroxy- und/oder 3-Alkoxybuttersäuren enthalten und welche durch das erfindungsgemäße Herstellungsverfahren erstmals in effizienter Weise zugänglich sind, stellen eine physiologisch und pharmakologisch relevante Alternative zu der freien 3-Hydroxybuttersäure bzw. deren Salzen dar.

Die Herstellung derartiger Verbindungen mittels herkömmlicher organischer Synthese ist komplex und aufwendig, da 3-Hydroxybuttersäure verstärkt zur Polymerisation und anderen unerwünschten Nebenreaktionen (z. B. Wasserabspaltung, Zersetzung etc.) neigt. Im Rahmen der vorliegenden Erfindung konnte erstmals ein effizient arbeitendes Herstellungsverfahren bereitgestellt werden, mit welchem sich Lipide, welche Struktureinheiten auf Basis von Glyceriden von 3-Hydroxy- und/oder 3-Alkoxybuttersäuren enthalten, ohne unerwünschte Nebenreaktionen herstellen lassen, insbesondere einstufig.

Das erfindungsgemäße Verfahren ermöglicht somit erstmalig die Bereitstellung untoxischer Lipide, welche Struktureinheiten auf Basis von Glyceriden von 3-Hydroxy- und/oder 3-Alkoxybuttersäuren enthalten, aus an sich bekannten, kommerziell verfügbaren und vor allem physiologisch unbedenklichen Komponenten bzw. Edukten (Ausgangsverbindungen). Die resultierenden Lipide, welche Struktureinheiten auf Basis von Glyceriden von 3-Hydroxy- und/oder 3-Alkoxybuttersäuren enthalten, können physiologisch, insbesondere im Magen und/oder im Darm, aufgespalten werden und das Zielmolekül "3-Hydroxybuttersäure" bzw. deren Salze als Wirkstoff bzw. Wirkkomponente freisetzen bzw. generieren.

Darüber hinaus weisen die vorgenannten Lipide, welche Struktureinheiten auf Basis von Glyceriden von 3-Hydroxy- und/oder 3-Alkoxybuttersäuren enthalten, auch einen akzeptablen Geschmack auf, um eine Kompatibilität auch bei oraler Verabreichung größerer Mengen über einen längeren Zeitraum zu gewährleisten (z. B. Verabreichung von 50 g Tagesdosis oder mehr).

Gleichermaßen ermöglicht es das erfindungsgemäße Herstellungsverfahren, die Lipide, welche Struktureinheiten auf Basis von Glyceriden von 3-Hydroxy- und/oder 3-Alkoxybuttersäuren enthalten, frei von toxischen Verunreinigungen bereitzustellen.

Darüber hinaus kann bei entsprechenden Ausgangsmaterialien die Herstellung auch enantioselektiv durchgeführt werden. So ermöglicht es beispielsweise das erfindungsgemäße Herstellungsverfahren, die biologisch relevante Form, d. h. das (R)-Enantiomer anzureichern, insbesondere durch Enzymkatalyse, um bei oraler Verabreichung das renale System von Patienten nicht zu belasten (d. h. Elimination über die Nieren). Grundsätzlich ist es aber auch möglich und kann es unter bestimmten Voraussetzungen zweckdienlich sein, das (S)-Enantiomer anzureichern.

Darüber hinaus ist das erfindungsgemäße Herstellungsverfahren, einschließlich optionaler Weiterverarbeitungs- bzw. Aufreinigungsverfahrensschritte, wirtschaftlich bzw. ökonomisch betreibbar und auch großtechnisch umsetzbar.

Insbesondere verwendet das erfindungsgemäße Herstellungsverfahren kommerziell verfügbare Edukte und ermöglicht darüber hinaus eine relativ einfache Verfahrensführung auch bei großtechnischer Umsetzung.

Im Gegensatz zu herkömmlichen Herstellungsverfahren des Standes der Technik kommt das erfindungsgemäße Herstellungsverfahren ohne komplexe Edukte aus und verläuft nur einstufig. Dennoch werden im Rahmen des erfindungsgemäßen Herstellungsverfahrens exzellente Ausbeuten erzielt, wobei in gleicher Weise die Bildung von Nebenprodukten minimiert bzw. vermieden wird.

Darüber hinaus ist das erfindungsgemäße Verfahren einfach und wirtschaftlich. Insbesondere wird das erfindungsgemäße Verfahren in Abwesenheit von Lösemitteln und/oder ohne jedwedes Lösemittel durchgeführt (d. h. also als Reaktion in Masse bzw. als Reaktion in Substanz bzw. als sogenannte *Bulk Reaction*); folglich sind die erhaltenen Reaktionsprodukte nicht mit Lösemittel verunreinigt und es muss kein Lösemittel nach Durchführung des Verfahrens bzw. der Reaktion aufwendig und energieträchtig entfernt und entsorgt bzw. rezykliert werden. Es werden zudem auch keine toxischen Nebenprodukte gebildet.

Was die Ausgangsverbindungen bzw. Edukt der allgemeinen Formel (I) anbelangt, so ist hierzu Folgendes auszuführen:
Gemäß einer besonderen Ausführungsform des erfindungsgemäßen Verfahrens kann es vorgesehen sein, dass in der allgemeinen Formel (I) die Reste R¹, R² und R³, gleich oder verschieden, jeweils unabhängig voneinander
   - Wasserstoff,
   - einen Rest vom Typ lineares (geradkettiges) oder verzweigtes, gesättigtes oder ein- oder mehrfach ungesättigtes C₁-C₁₁-Alkyl-C(O)-, insbesondere lineares (geradkettiges) oder verzweigtes, gesättigtes oder ein- oder mehrfach ungesättigtes C₂-C₁₁-Alkyl-C(O)-,
   - einen Rest vom Typ lineares (geradkettiges) oder verzweigtes, gesättigtes oder ein- oder mehrfach ungesättigtes C₁₂-C₂₉-Alkyl-C(O)-, insbesondere lineares (geradkettiges) oder verzweigtes, gesättigtes oder ein- oder mehrfach ungesättigtes C₁₉-C₂₉-Alkyl-C(O)-,
darstellen, jedoch mit der Maßgabe, dass mindestens einer, vorzugsweise mindestens zwei der Reste R¹, R² und R³ keinen Wasserstoff darstellen.
Gemäß einer weiteren besonderen Ausführungsform des erfindungsgemäßen Verfahrens kann es vorgesehen sein, dass in der allgemeinen Formel (I) die Reste R¹, R² und R³, gleich oder verschieden, jeweils unabhängig voneinander
   - einen Rest vom Typ lineares (geradkettiges) oder verzweigtes, gesättigtes oder ein- oder mehrfach ungesättigtes C₁-C₁₁-Alkyl-C(O)-, insbesondere lineares (geradkettiges) oder verzweigtes, gesättigtes oder ein- oder mehrfach ungesättigtes C₂-C₁₁-Alkyl-C(O)-,
   - einen Rest vom Typ lineares (geradkettiges) oder verzweigtes, gesättigtes oder ein- oder mehrfach ungesättigtes C₁₂-C₂₉-Alkyl-C(O)-, insbesondere lineares (geradkettiges) oder verzweigtes, gesättigtes oder ein- oder mehrfach ungesättigtes C₁₉-C₂₉-Alkyl-C(O)-,
darstellen.
Gemäß einer wiederum weiteren besonderen Ausführungsform des erfindungsgemäßen Verfahrens kann es vorgesehen sein, dass in der allgemeinen Formel (I) die Reste R¹, R² und R³, gleich oder verschieden, jeweils unabhängig voneinander
   - einen Rest vom Typ lineares (geradkettiges) oder verzweigtes, gesättigtes oder ein- oder mehrfach ungesättigtes C₁-C₁₁-Alkyl-C(O)-, insbesondere lineares (geradkettiges) oder verzweigtes, gesättigtes oder ein- oder mehrfach ungesättigtes C₂-C₁₁-Alkyl-C(O)-,
   - einen Rest vom Typ lineares (geradkettiges) oder verzweigtes, gesättigtes oder ein- oder mehrfach ungesättigtes C₁₂-C₂₉-Alkyl-C(O)-, insbesondere lineares (geradkettiges) oder verzweigtes, gesättigtes oder ein- oder mehrfach ungesättigtes C₁₉-C₂₉-Alkyl-C(O)-,
darstellen, jedoch mit der Maßgabe, dass mindestens einer der Reste R¹, R² und R³ einen Rest vom Typ lineares (geradkettiges) oder verzweigtes, gesättigtes oder ein-oder mehrfach ungesättigtes C₁-C₁₁-Alkyl-C(O)-, insbesondere lineares (geradkettiges) oder verzweigtes, gesättigtes oder ein- oder mehrfach ungesättigtes C₂-C₁₁-Alkyl-C(O)-, darstellt.
Gemäß einer wiederum noch weiteren besonderen Ausführungsform des erfindungsgemäßen Verfahrens kann es vorgesehen sein, dass in der allgemeinen Formel (I) die Reste R¹, R² und R³, gleich oder verschieden, jeweils unabhängig voneinander
   - einen Rest vom Typ lineares (geradkettiges) oder verzweigtes, gesättigtes oder ein- oder mehrfach ungesättigtes C₁-C₁₁-Alkyl-C(O)-, insbesondere lineares (geradkettiges) oder verzweigtes, gesättigtes oder ein- oder mehrfach ungesättigtes C₂-C₁₁-Alkyl-C(O)-,
   - einen Rest vom Typ lineares (geradkettiges) oder verzweigtes, gesättigtes oder ein- oder mehrfach ungesättigtes C₁₂-C₂₉-Alkyl- C(O) -, insbesondere lineares (geradkettiges) oder verzweigtes, gesättigtes oder ein- oder mehrfach ungesättigtes C₁₉-C₂₉-Alkyl-C(O)-,
darstellen, jedoch mit der Maßgabe, dass mindestens einer der Reste R¹, R² und R³ einen Rest vom Typ lineares (geradkettiges) oder verzweigtes, gesättigtes oder ein-oder mehrfach ungesättigtes C₁₂-C₂₉-Alkyl-C(O)-, insbesondere lineares (geradkettiges) oder verzweigtes, gesättigtes oder ein- oder mehrfach ungesättigtes C₁₉-C₂₉-Alkyl-C(O)- , darstellt.
Schließlich kann es gemäß einer wiederum noch weiteren besonderen Ausführungsform des erfindungsgemäßen Verfahrens vorgesehen sein, dass in der allgemeinen Formel (I) die Reste R¹, R² und R³, gleich oder verschieden, jeweils unabhängig voneinander
   - einen Rest vom Typ lineares (geradkettiges) oder verzweigtes, gesättigtes oder ein- oder mehrfach ungesättigtes C₁-C₁₁-Alkyl-C(O)-, insbesondere lineares (geradkettiges) oder verzweigtes, gesättigtes oder ein- oder mehrfach ungesättigtes 6₂-C₁₁-Alkyl-C(O)-,
   - einen Rest vom Typ lineares (geradkettiges) oder verzweigtes, gesättigtes oder ein- oder mehrfach ungesättigtes C₁₂-C₂₉-Alkyl-C(O)-, insbesondere lineares (geradkettiges) oder verzweigtes, gesättigtes oder ein- oder mehrfach ungesättigtes C₁₉-C₂₉-Alkyl-C(O)-,
darstellen, jedoch mit der Maßgabe, dass mindestens einer der Reste R¹, R² und R³ einen Rest vom Typ lineares (geradkettiges) oder verzweigtes, gesättigtes oder ein-oder mehrfach ungesättigtes C₁-C₁₁-Alkyl-C(O)-, insbesondere lineares (geradkettiges) oder verzweigtes, gesättigtes oder ein- oder mehrfach ungesättigtes C₂-C₁₁-Alkyl-C(O)-, darstellt, und mit der Maßgabe, dass mindestens einer der Reste R¹, R² und R³ einen Rest vom Typ lineares (geradkettiges) oder verzweigtes, gesättigtes oder ein- oder mehrfach ungesättigtes C₁₂-C₂₉-Alkyl-C(O)-, insbesondere lineares (geradkettiges) oder verzweigtes, gesättigtes oder ein- oder mehrfach ungesättigtes C₁₉-C₂₉-Alkyl- C(O) - , darstellt.

Insbesondere kann es vorgesehen sein, dass in der allgemeinen Formel (I) die Reste R¹, R² und R³, gleich oder verschieden, jeweils unabhängig voneinander einen Rest vom Typ lineares (geradkettiges) oder verzweigtes, gesättigtes oder ein-oder mehrfach ungesättigtes C₁-C₁₁-Alkyl-C(O)-, insbesondere lineares (geradkettiges) oder verzweigtes, gesättigtes oder ein- oder mehrfach ungesättigtes C₂-C₁₁-Alkyl-C(O)-, darstellen.

Weiterhin kann es auch vorgesehen sein, dass in der allgemeinen Formel (I) die Reste R¹, R² und R³, gleich oder verschieden, jeweils unabhängig voneinander einen Rest vom Typ lineares (geradkettiges) oder verzweigtes, gesättigtes oder ein-oder mehrfach ungesättigtes C₁₂-C₂₉-Alkyl-C(O)-, insbesondere lineares (geradkettiges) oder verzweigtes, gesättigtes oder ein- oder mehrfach ungesättigtes C₁₉-C₂₉-Alkyl-C(O)-, darstellen.

Was die Ausgangsverbindungen bzw. Edukt der allgemeinen Formel (II) anbelangt, so ist hierzu Folgendes auszuführen:
Gemäß einer besonderen Ausführungsform des erfindungsgemäßen Verfahrens kann es vorgesehen sein, dass in der allgemeinen Formel (II)
   - der Rest R⁴ Wasserstoff oder einen Rest vom Typ lineares (geradkettiges) oder verzweigtes, gesättigtes oder ein- oder mehrfach ungesättigtes C₁-C₃₀-Alkyl-C(O)-, insbesondere lineares (geradkettiges) oder verzweigtes, gesättigtes oder ein- oder mehrfach ungesättigtes C₁-C₂₁-Alkyl-C(O)-, vorzugsweise lineares (geradkettiges) oder verzweigtes, gesättigtes oder ein- oder mehrfach ungesättigtes C₃-C₂₁-Alkyl-C(O)-, darstellt,
   - der Rest R⁵ ein C₁-C₄-Alkyl, insbesondere ein C₁-C₄-Alkyl, bevorzugt Methyl oder Ethyl, besonders bevorzugt Ethyl, darstellt.

Gemäß einer weiteren besonderen Ausführungsform des erfindungsgemäßen Verfahrens kann es vorgesehen sein, dass in der allgemeinen Formel (II)
- der Rest R⁴ Wasserstoff oder einen Rest vom Typ lineares (geradkettiges) oder verzweigtes, gesättigtes oder ein- oder mehrfach ungesättigtes C₁-C₃₀-Alkyl-C(O)-, insbesondere lineares (geradkettiges) oder verzweigtes, gesättigtes oder ein- oder mehrfach ungesättigtes C₁-C₂₁-Alkyl-C(O)-, vorzugsweise lineares (geradkettiges) oder verzweigtes, gesättigtes oder ein- oder mehrfach ungesättigtes C₃-C₂₁-Alkyl-C(O)-, darstellt,
- der Rest R⁵ Ethyl darstellt.

Gemäß einer wiederum weiteren besonderen Ausführungsform des erfindungsgemäßen Verfahrens kann es vorgesehen sein, dass wobei in der allgemeinen Formel (II) der Rest R⁴ Wasserstoff und der Rest R⁵ Ethyl darstellt.

Insbesondere kann als 3-Hydroxy- und/oder 3-Alkoxybuttersäure-Derivat der allgemeinen Formel (II) 3-Hydroxybuttersäureethylester (Ethyl-3-hydroxybutyrat) der Formel CH₃-CH(OH)-CH₂-C(O)OC₂H₅ eingesetzt werden.

Grundsätzlich kann das 3-Hydroxy- und/oder 3-Alkoxybuttersäure-Derivat der allgemeinen Formel (II) in racemischer Form oder in Form des (R)-Enantiomers eingesetzt werden. Dabei ist die (R)-Konfiguration insbesondere auf das Kohlenstoffatom in 3-Position bezogen ist bzw. ist die (R)-Konfiguration auf das den OR⁴-Rest tragende Kohlenstoffatom bezogen.

Bei dem erfindungsgemäßen Verfahren wird die Umsetzung in Abwesenheit von Lösemitteln und/oder ohne jedwedes Lösemittel durchgeführt. D. h. die Umsetzung wird also als Reaktion in Masse bzw. als Reaktion in Substanz bzw. als sogenannte *Bulk Reaction* durchgeführt. Dies hat den Vorteil, dass die erhaltenen Reaktionsprodukte nicht mit Lösemittel verunreinigt sind und kein Lösemittel nach Durchführung des Verfahrens bzw. der Reaktion aufwendig und energieträchtig entfernt und entsorgt bzw. rezykliert werden muss. Überraschenderweise verläuft das Verfahren bzw. die Reaktion dennoch mit hohen Umsätzen und Ausbeuten und zumindest im Wesentlichen ohne signifikante Nebenproduktbildung.

Erfindungsgemäß ist vorgesehen, dass die Umsetzung in Gegenwart eines Enzyms als Katalysator durchgeführt wird. Dabei kann der Katalysators nach Umsetzung insbesondere rezykliert werden.

Gemäß dem erfindungsgemäßen Verfahren wird die Umsetzung in Gegenwart eines Enzyms als Katalysator durchgeführt.

Dabei kann das Enzym insbesondere ausgewählt werden aus Synthetasen (Ligasen), Katalasen, Esterasen, Lipasen und deren Kombinationen.

Insbesondere kann sich dabei das Enzym ableiten von *Candida antarctica, Mucor miehei (Rhizomucor miehei), Thermomyces lanuginosus, Candida rugosa, Aspergillus oryzae, Pseudomonas cepacia, Pseudomonas fluorescens, Rhizopus delemar* und *Pseudomonas* sp. sowie deren Kombinationen, vorzugsweise von *Candida antarctica, Mucor miehei (Rhizomucor miehei)* und *Thermomyces lanuginosus.*

Insbesondere kann das Enzym in immobilisierter Form, insbesondere immobilisiert auf einem Träger, vorzugsweise auf einem polymeren Träger, bevorzugt auf einem polymeren organischen Träger, besonders bevorzugt mit hydrophoben Eigenschaften, ganz besonders bevorzugt auf einem poly(meth)acrylharzbasierten Träger, eingesetzt werden.

Insbesondere kann das Enzym nach der Umsetzung rezykliert werden.

Erfindungsgemäß wird die Umsetzung in Gegenwart eines Enzyms als Katalysator durchgeführt; dabei kann die Umsetzung in Gegenwart eines Enzyms als Katalysator insbesondere bei Temperaturen im Bereich von 10 °C bis 80 °C, insbesondere im Bereich von 20 °C bis 80 °C, vorzugsweise im Bereich von 25 °C bis 75 °C, besonders bevorzugt im Bereich von 45 °C bis 75 °C, ganz besonders bevorzugt im Bereich von 50 °C bis 70 °C, durchgeführt werden.

Das Enzym kann insbesondere in Mengen, bezogen auf die Gesamtmenge der Ausgangsverbindungen (I) und (II), im Bereich von 0,001 Gew.-% bis 20 Gew.-%, insbesondere im Bereich von 0,01 Gew.-% bis 15 Gew.-%, vorzugsweise im Bereich von 0,1 Gew.-% bis 15 Gew.-%, bevorzugt im Bereich von 0,5 Gew.-% bis 10 Gew.-%, eingesetzt werden.

Die Umsetzung in Gegenwart eines Enzyms als Katalysator kann insbesondere bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt werden.

Bezüglich der Herstellung der Ausgangsverbindungen bzw. Edukte ist Folgendes auszuführen:
Für den Fall, dass in der allgemeinen Formel (I) mindestens einer der Reste R¹, R² und R³ einen Rest C₁-C₁₁-Alkyl-C(O)-, insbesondere C₂-C₁₁-Alkyl-C(O)-, darstellt und mindestens einer der Reste R¹, R² und R³ einen Rest C₁₂-C₂₉-Alkyl-C(O)-, insbesondere C₁₉-C₂₉-Alkyl-C(O)-, darstellt, ist das Glycerid der allgemeinen Formel (I) durch entsprechende Umesterung erhältlich bzw. kann das Glycerid der allgemeinen Formel (I) durch entsprechende Umesterung erhalten werden.

Gemäß einer besonderen Ausführungsform des erfindungsgemäßen Verfahrens kann die Umesterung dadurch durchgeführt werden, dass mindestens eine Verbindung der allgemeinen Formel (la)

R⁹O-CH₂-CH(OR¹⁰)-CH₂-OR¹¹ (Ia)

wobei in der allgemeinen Formel (la) die Reste R⁹, R¹⁰ und R¹¹, gleich oder verschieden, jeweils unabhängig voneinander
   - Wasserstoff,
   - einen Rest C₁-C₁₁-Alkyl-C(O)-, insbesondere C₂-C₁₁-Alkyl-C(O)-,
darstellen, jedoch mit der Maßgabe, dass mindestens zwei und vorzugsweise der Reste R⁹, R¹⁰ und R¹¹ keinen Wasserstoff darstellen,
mit mindestens einer Verbindung der allgemeinen Formel (Ib)

R¹²O-CH₂-CH(OR¹³)-CH₂-OR¹⁴ (Ib)

wobei in der allgemeinen Formel (Ib) die Reste R¹², R¹³ und R¹⁴, gleich oder verschieden, jeweils unabhängig voneinander
   - Wasserstoff,
   - einen Rest C₁₂-C₂₉-Alkyl-C(O)-, insbesondere C₁₉-C₂₉-Alkyl-C(O)-,
darstellen, jedoch mit der Maßgabe, dass mindestens zwei und vorzugsweise der Reste R¹², R¹³ und R¹⁴ keinen Wasserstoff darstellen,

unter Umesterungsbedingungen umgesetzt wird
oder aber (umgekehrt) mindestens eine Verbindung der allgemeinen Formel (Ib), wie zuvor definiert, mit mindestens einer Verbindung der allgemeinen Formel (la), wie zuvor definiert, unter Umesterungsbedingungen umgesetzt wird.

Dabei können in der allgemeinen Formel (la) die Reste R⁹, R¹⁰ und R¹¹, gleich oder verschieden, jeweils unabhängig voneinander insbesondere einen Rest vom Typ lineares (geradkettiges) oder verzweigtes, gesättigtes oder ein- oder mehrfach ungesättigtes C₁-C₁₁-Alkyl-C(O)-, insbesondere lineares (geradkettiges) oder verzweigtes, gesättigtes oder ein- oder mehrfach ungesättigtes C₂-C₁₁-Alkyl-C(O)-, darstellen.

Weiterhin können in der allgemeinen Formel (Ib) die Reste R¹², R¹³ und R¹⁴, gleich oder verschieden, jeweils unabhängig voneinander insbesondere einen Rest vom Typ lineares (geradkettiges) oder verzweigtes, gesättigtes oder ein- oder mehrfach ungesättigtes C₁₂-C₂₉-Alkyl-C(O)-, insbesondere lineares (geradkettiges) oder verzweigtes, gesättigtes oder ein- oder mehrfach ungesättigtes C₁₉-C₂₉-Alkyl-C(O) - , darstellen.

Insbesondere kann es erfindungsgemäß vorgesehen sein, dass für den Fall, dass in der allgemeinen Formel (la) einer der Reste R⁹, R¹⁰ und R¹¹ Wasserstoff darstellt, die Verbindung der allgemeinen Formel (la) erhältlich ist und/oder erhalten wird durch partielle Hydrolyse, insbesondere partielle (selektiv) enzymatisch katalysierte Hydrolyse eines betreffenden Ausgangs-Triglycerids der allgemeinen Formel (la), bei welchem keiner der Reste R⁹, R¹⁰ und R¹¹ Wasserstoff darstellt und/oder bei welchem Reste R⁹, R¹⁰ und R¹¹, gleich oder verschieden, jeweils unabhängig voneinander einen Rest C₁-C₁₁-Alkyl-C(O)-, insbesondere C₂-C₁₁-Alkyl-C(O)-, darstellen.

Weiterhin kann es erfindungsgemäß insbesondere vorgesehen sein, dass in der allgemeinen Formel (Ib) einer der Reste R¹², R¹³ und R¹⁴ Wasserstoff darstellt, die Verbindung der allgemeinen Formel (Ib) erhältlich ist und/oder erhalten wird durch partielle Hydrolyse, insbesondere partielle (selektiv) enzymatisch katalysierte Hydrolyse eines betreffenden Ausgangs-Triglycerids der allgemeinen Formel (Ib), bei welchem keiner der Reste R¹², R¹³ und R¹⁴ Wasserstoff darstellt und/oder bei welchem Reste R¹², R¹³ und R¹⁴, gleich oder verschieden, jeweils unabhängig voneinander einen Rest C₁₂-C₂₉-Alkyl-C(O)-, insbesondere C₁₉-C₂₉-Alkyl-C(O)-, darstellen.

Gemäß einer besonderen Ausführungsform des erfindungsgemäßen Verfahrens kann es vorgesehen sein, dass die Umesterung enzymkatalytisch durchgeführt wird.

Gemäß dieser besonderen Ausführungsform des erfindungsgemäßen Verfahrens, wonach die Umesterung in Gegenwart eines Enzyms als Katalysator durchgeführt wird, kann das Enzym insbesondere ausgewählt werden aus Synthetasen (Ligasen), Katalasen, Esterasen, Lipasen und deren Kombinationen. Insbesondere kann sich das Enzym ableiten von *Candida antarctica, Mucor miehei* (*Rhizomucor miehei*)*, Thermomyces lanuginosus, Candida rugosa, Aspergillus oryzae, Pseudomonas cepacia, Pseudomonas fluorescens, Rhizopus delemar* und *Pseudomonas* sp. sowie deren Kombinationen, vorzugsweise von *Candida antarctica, Mucor miehei* (*Rhizomucor miehei*) und *Thermomyces lanuginosus.* Insbesondere kann dabei das Enzym in immobilisierter Form, insbesondere immobilisiert auf einem Träger, vorzugsweise auf einem polymeren Träger, bevorzugt auf einem polymeren organischen Träger, besonders bevorzugt mit hydrophoben Eigenschaften, ganz besonders bevorzugt auf einem poly(meth)acrylharzbasierten Träger, eingesetzt werden. Bevorzugt kann das Enzym nach der Umesterung rezykliert werden.

Insbesondere kann dabei die Umesterung in Gegenwart eines Enzyms als Katalysator bei Temperaturen im Bereich von 10 °C bis 80 °C, insbesondere im Bereich von 20 °C bis 80 °C, vorzugsweise im Bereich von 25 °C bis 75 °C, besonders bevorzugt im Bereich von 45 °C bis 75 °C, ganz besonders bevorzugt im Bereich von 50 °C bis 70 °C, durchgeführt werden.

Insbesondere kann das Enzym in Mengen, bezogen auf die Gesamtmenge der Ausgangsverbindungen (la) und (Ib), im Bereich von 0,001 Gew.-% bis 20 Gew.-%, insbesondere im Bereich von 0,01 Gew.-% bis 15 Gew.-%, vorzugsweise im Bereich von 0,1 Gew.-% bis 15 Gew.-%, bevorzugt im Bereich von 0,5 Gew.-% bis 10 Gew.-%, eingesetzt werden.

Dabei kann die Umesterung in Gegenwart eines Enzyms als Katalysator insbesondere bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt werden.

Gemäß einer besonderen Ausführungsform des erfindungsgemäßen Verfahrens kann es vorgesehen sein, dass für den Fall, dass in der allgemeinen Formel (II) der Rest R⁴ einen Rest C₁-C₃₀-Alkyl-C(O)-, insbesondere C₁-C₂₁-Alkyl-C(O)-, vorzugsweise C₃-C₂₁-Alkyl-C(O)-, darstellt, das 3-Hydroxy- und/oder 3-Alkoxybuttersäure-Derivat der allgemeinen Formel (II) erhältlich ist und/oder erhalten wird durch Umsetzung einer Verbindung der allgemeinen Formel (IV)

CH₃-CH(OH)-CH₂-C(O)OR⁵ (IV)

wobei in der allgemeinen Formel (IV) der Rest R⁵ die zuvor angegebene Bedeutung hat, insbesondere Wasserstoff oder ein C₁-C₄-Alkyl, insbesondere ein C₁-C₄-Alkyl, bevorzugt Methyl oder Ethyl, besonders bevorzugt Ethyl, darstellt,
mit mindestens einem Carbonsäureanhydrid der allgemeinen Formel (V)

   R¹⁵-C(O)-O-C(O)-R¹⁶ (V)

   wobei in der in der allgemeinen Formel (V) die Reste R¹⁵ und R¹⁶, gleich oder verschieden, jeweils unabhängig voneinander einen Rest C₁-C₃₀-Alkyl-C(O)-, insbesondere C₁-C₂₁-Alkyl-C(O)-, vorzugsweise C₃-C₂₁-Alkyl-C(O)-, darstellen,
gegebenenfalls gefolgt von einer Hydrolyse für den Fall, dass R⁵ Wasserstoff darstellt.

Insbesondere kann dabei die Umsetzung der mindestens einen Verbindung der allgemeinen Formel (IV) mit dem mit mindestens einen Carbonsäureanhydrid der allgemeinen Formel (V) bei Temperaturen im Bereich von 60 bis 150°C, insbesondere im Bereich von 70 bis 120 °C, vorzugsweise im Bereich von 80 bis 100 °C, durchgeführt werden.

Weiterhin kann die Umsetzung der mindestens einen Verbindung der allgemeinen Formel (IV) mit dem mit mindestens einen Carbonsäureanhydrid der allgemeinen Formel (V) bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt werden.

Insbesondere wird bei der Umsetzung der mindestens einen Verbindung der allgemeinen Formel (IV) mit dem mit mindestens einen Carbonsäureanhydrid der allgemeinen Formel (V) gleichzeitig eine Verbindung gemäß der allgemeinen Formel (VI)

R¹⁷-C(O)-OH (VI)

wobei der Rest R¹⁷ einen Rest R¹⁵ oder R¹⁶ jeweils mit der zuvor angegebenen Bedeutung darstellt, gebildet. Insbesondere kann die Verbindung gemäß der allgemeinen Formel (VI) während oder nach erfolgter Umsetzung, insbesondere nach erfolgter Umsetzung, entfernt werden, vorzugsweise destillativ.

Gemäß einer besonderen Ausführungsform kann es vorgesehen sein, dass für den Fall, dass in der allgemeinen Formel (V) die Reste R¹⁵ und R¹⁶ voneinander verschieden sind, und/oder für den Fall, dass in der allgemeinen Formel (V) die Reste R¹⁵ und R¹⁶ jeweils einen Alkylrest mit mehr als zwei Kohlenstoffatomen darstellen, das Carbonsäureanhydrid der allgemeinen Formel (V) erhältlich ist und/oder erhalten wird durch Umsetzung von Essigsäureanhydrid (Acetanhydrid) mit mindestens einer Carbonsäure der allgemeinen Formel (VII)

R¹⁸- C(O)-OH (VII)

wobei der Rest R¹⁸ einen Rest R¹⁵ oder R¹⁶ jeweils mit der zuvor angegebenen Bedeutung darstellt, jedoch mit der Maßgabe, dass die Reste R¹⁵ und R¹⁶ voneinander verschieden sind und/oder dass die Reste R¹⁵ und R¹⁶, gleich oder verschieden, jeweils unabhängig voneinander einen Alkylrest mit mehr als zwei Kohlenstoffatomen darstellen.

Insbesondere erfolgt die Umsetzung von Essigsäureanhydrid (Acetanhydrid) mit der mindestens einen Carbonsäure der allgemeinen Formel (VII) entsprechend der Reaktionsgleichung

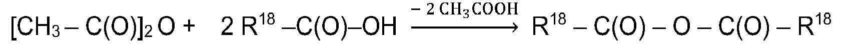

Dabei kann die Umsetzung von Essigsäureanhydrid (Acetanhydrid) mit der mindestens einen Carbonsäure der allgemeinen Formel (VII) bei Temperaturen im Bereich von 60 bis 150 °C, insbesondere im Bereich von 70 bis 120 °C, vorzugsweise im Bereich von 80 bis 100 °C, durchgeführt werden.

Die Umsetzung von Essigsäureanhydrid (Acetanhydrid) mit der mindestens einen Carbonsäure der allgemeinen Formel (VII) kann insbesondere bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt werden.

Nach dieser Synthese kann insbesondere ein symmetrisches Carbonsäureanhydrid der allgemeinen Formel (V) hergestellt werden. Insbesondere können in der allgemeinen Formel (V) die Reste R¹⁵ und R¹⁶ identisch sein und einen Alkylrest mit mehr als zwei Kohlenstoffatomen darstellen.

Gemäß einer alternativen Ausführungsform kann nach dieser Synthese aber auch ein unsymmetrisches Carbonsäureanhydrid der allgemeinen Formel (V) hergestellt werden. Insbesondere können in der allgemeinen Formel (V) die Reste R¹⁵ und R¹⁶ voneinander verschieden sein. Vorzugsweise können dabei in der allgemeinen Formel (V) die Reste R¹⁵ und R¹⁶ jeweils einen Alkylrest mit mehr als zwei Kohlenstoffatomen darstellen.

Im Rahmen des erfindungsgemäßen Herstellungsverfahrens kann für den Fall, dass in der allgemeinen Formel (II) der Rest R⁵ Wasserstoff bezeichnet, anstelle der freien Säure deren Anhydrid der allgemeinen Formel (IIa)

[CH₃-CH(OR⁴)-CH₂-C(O)]₂O (IIa)

wobei in der allgemeinen Formel (IIa) der Rest R⁴ die zuvor angegebene Bedeutung hat,
als Ausgangsverbindung bzw. Edukt (II) eingesetzt werden.

Im Rahmen des erfindungsgemäßen Herstellungsverfahrens wird als Reaktionsprodukt ein Gemisch von mindestens zwei voneinander verschiedenen, Struktureinheiten auf Basis von Glyceriden von 3-Hydroxy- und/oder 3-Alkoxybuttersäuren enthaltenden Lipiden der allgemeinen Formel (III)

R⁶O-CH₂-CH(OR⁷)-CH₂-OR⁸ (III)

wobei in der allgemeinen Formel (III) die Reste R⁶, R⁷ und R⁸, gleich oder verschieden, jeweils unabhängig voneinander
   - einen Rest C₁-C₁₁-Alkyl-C(O)- , insbesondere C₂-C₁₁-Alkyl-C(O)-,
   - einen Rest C₁₂-C₂₉-Alkyl-C(O)-, insbesondere C₁₉-C₂₉-Alkyl-C(O)-,
   - einen Rest CH₃-CH(OR⁴)-CH₂-C(O)-, worin der Rest R⁴ die zuvor angegebene Bedeutung hat, insbesondere wobei der Rest R⁴ Wasserstoff oder einen Rest C₁-C₃₀-Alkyl-C(O)-, insbesondere C₁-C₂₁-Alkyl-C(O)-, vorzugsweise C₃-C₂₁-Alkyl-C(O)-, darstellt,
darstellen, jedoch mit der Maßgabe, dass einer oder zwei der Reste R⁶, R⁷ und R⁸ einen Rest CH₃-CH(OR⁴)-CH₂-C(O)-darstellt (und vorzugsweise mit der weiteren Maßgabe dass für den Fall, dass alle Reste R⁶, R⁷ und R⁸ einen Rest CH₃-CH(OR⁴)-CH₂-C(O)-darstellen, bei mindestens einem der Reste R⁶, R⁷ und R⁸ im Rest CH₃-CH(OR⁴)-CH₂-C(O)- der Rest R⁴ keinen Wasserstoff darstellt),
erhalten.

Insbesondere kann im Rahmen des erfindungsgemäßen Herstellungsverfahrens als Reaktionsprodukt ein Gemisch von mindestens zwei voneinander verschiedenen, Struktureinheiten auf Basis von Glyceriden von 3-Hydroxy- und/oder 3-Alkoxybuttersäuren enthaltenden Lipiden der allgemeinen Formel (III)

R⁶O-CH₂-CH(OR⁷)-CH₂-OR⁸ (III)

wobei in der allgemeinen Formel (III) die Reste R⁶, R⁷ und R⁸, gleich oder verschieden, jeweils unabhängig voneinander
   - einen Rest vom Typ lineares (geradkettiges) oder verzweigtes, gesättigtes oder ein- oder mehrfach ungesättigtes C₁-C₁₁-Alkyl-C(O)-, insbesondere lineares (geradkettiges) oder verzweigtes, gesättigtes oder ein- oder mehrfach ungesättigtes C₂-Cn-Alkyl-C(O)-,
   - einen Rest vom Typ lineares (geradkettiges) oder verzweigtes, gesättigtes oder ein- oder mehrfach ungesättigtes C₁₂-C₂₉-Alkyl-C(O)-, insbesondere lineares (geradkettiges) oder verzweigtes, gesättigtes oder ein- oder mehrfach ungesättigtes C₁₉-C₂₉-Alkyl-C(O)-,
   - einen Rest vom Typ lineares (geradkettiges) oder verzweigtes, gesättigtes oder ein- oder mehrfach ungesättigtes CH₃-CH(OR⁴)-CH₂-C(O)-, worin der Rest R⁴ die zuvor angegebene Bedeutung hat, insbesondere wobei der Rest R⁴ Wasserstoff oder einen Rest C₁-C₃₀-Alkyl-C(O)-, insbesondere C₁-C₂₁-Alkyl-C(O)-, vorzugsweise C₃-C₂₁-Alkyl-C(O)-, darstellt,
darstellen, jedoch mit der Maßgabe, dass einer oder zwei der Reste R⁶, R⁷ und R⁸ einen Rest CH₃-CH(OR⁴)-CH₂-C(O)-darstellt.
erhalten werden.

Insbesondere kann im Rahmen des erfindungsgemäßen Herstellungsverfahrens als Reaktionsprodukt ein Gemisch von mindestens zwei voneinander verschiedenen, Struktureinheiten auf Basis von Glyceriden von 3-Hydroxy- und/oder 3-Alkoxybuttersäuren enthaltenden Lipiden der allgemeinen Formel (III)

R⁶O-CH₂-CH(OR⁷)-CH₂-OR⁸ (III)

wobei in der allgemeinen Formel (III) die Reste R⁶, R⁷ und R⁸, gleich oder verschieden, jeweils unabhängig voneinander
   - einen Rest C₁-C₁₁-Alkyl-C(O)-, insbesondere C₂-C₁₁-Alkyl-C(O)-,
   - einen Rest C₁₂-C₂₉-Alkyl-C(O)-, insbesondere C₁₉-C₂₉-Alkyl-C(O)-,
   - einen Rest CH₃-CH(OR⁴)-CH₂-C(O)- , worin der Rest R⁴ die zuvor angegebene Bedeutung hat, insbesondere wobei der Rest R⁴ Wasserstoff oder einen Rest C₁-C₃₀-Alkyl-C(O)-, insbesondere C₁-C₂₁-Alkyl-C(O)-, vorzugsweise C₃-C₂₁-Alkyl-C(O)-, darstellt,
darstellen, jedoch mit der Maßgabe, dass zwei Reste R⁶, R⁷ und R⁸ einen Rest CH₃-CH(OR⁴)-CH₂-C(O)- darstellen,
erhalten werden.

Gemäß einer besonderen Ausführungsform des erfindungsgemäßen Herstellungsverfahrens kann als Reaktionsprodukt ein Gemisch von mindestens zwei voneinander verschiedenen, Struktureinheiten auf Basis von Glyceriden von 3-Hydroxy- und/oder 3-Alkoxybuttersäuren enthaltenden Lipiden der allgemeinen Formel (III)

R⁶O-CH₂-CH(OR⁷)-CH₂-OR⁸ (III)

wobei in der allgemeinen Formel (III) die Reste R⁶, R⁷ und R⁸, gleich oder verschieden, jeweils unabhängig voneinander
   - einen Rest C₁-C₁₁-Alkyl-C(O)-, insbesondere C₂-C₁₁-Alkyl-C(O)-,
   - einen Rest CH₃-CH(OR⁴)-CH₂-C(O)-, worin der Rest R⁴ die zuvor angegebene Bedeutung hat, insbesondere wobei der Rest R⁴ Wasserstoff oder einen Rest C₁-C₃₀-Alkyl-C(O)-, insbesondere C₁-C₂₁-Alkyl-C(O)-, vorzugsweise C₃-C₂₁-Alkyl-C(O)-, darstellt,
darstellen, jedoch mit der Maßgabe, dass zwei Reste R⁶, R⁷ und R⁸ einen Rest CH₃-CH(OR⁴)-CH₂-C(O)- darstellen,
erhalten werden.

Gemäß einer weiteren besonderen Ausführungsform des erfindungsgemäßen Herstellungsverfahrens kann als Reaktionsprodukt ein Gemisch von mindestens zwei voneinander verschiedenen, Struktureinheiten auf Basis von Glyceriden von 3-Hydroxy- und/oder 3-Alkoxybuttersäuren enthaltenden Lipiden der allgemeinen Formel (III)

R⁶O-CH₂-CH(OR⁷)-CH₂-OR⁸ (III)

wobei in der allgemeinen Formel (III) die Reste R⁶, R⁷ und R⁸, gleich oder verschieden, jeweils unabhängig voneinander
   - einen Rest C₁₂-C₂₉-Alkyl-C(O)-, insbesondere C₁₉-C₂₉-Alkyl-C(O)-,
   - einen Rest CH₃-CH(OR⁴)-CH₂-C(O)-, worin der Rest R⁴ die zuvor angegebene Bedeutung hat, insbesondere wobei der Rest R⁴ Wasserstoff oder einen Rest C₁-C₃₀-Alkyl-C(O)-, insbesondere C₁-C₂₁-Alkyl-C(O)-, vorzugsweise C₃-C₂₁-Alkyl- C(O) - , darstellt,
darstellen, jedoch mit der Maßgabe, dass zwei Reste R⁶, R⁷ und R⁸ einen Rest CH₃-CH(OR⁴)-CH₂-C(O)- darstellen,
erhalten werden.

Gemäß einer wiederum weiteren besonderen Ausführungsform des erfindungsgemäßen Herstellungsverfahrens kann als Reaktionsprodukt ein Gemisch von mindestens zwei voneinander verschiedenen, Struktureinheiten auf Basis von Glyceriden von 3-Hydroxy- und/oder 3-Alkoxybuttersäuren enthaltenden Lipiden der allgemeinen Formel (III)

R⁶O-CH₂-CH(OR⁷)-CH₂-OR⁸ (III)

wobei in der allgemeinen Formel (III) die Reste R⁶, R⁷ und R⁸, gleich oder verschieden, jeweils unabhängig voneinander
   - einen Rest C₁-C₁₁-Alkyl-C(O)-, insbesondere C₂-C₁₁-Alkyl-C(O)-,
   - einen Rest C₁₂-C₂₉-Alkyl-C(O)-, insbesondere C₁₉-C₂₉-Alkyl-C(O)-,
   - einen Rest CH₃-CH(OR⁴)-CH₂-C(O)-, worin der Rest R⁴ die zuvor angegebene Bedeutung hat, insbesondere wobei der Rest R⁴ Wasserstoff oder einen Rest C₁-C₃₀-Alkyl-C(O)-, insbesondere C₁-C₂₁-Alkyl-C(O)-, vorzugsweise C₃-C₂₁-Alkyl-C(O)-, darstellt,
darstellen, jedoch mit der Maßgabe, dass einer der Reste R⁶, R⁷ und R⁸ einen Rest CH₃-CH(OR⁴)-CH₂-C(O)- darstellt,
erhalten werden.

Gemäß einer wiederum noch weiteren besonderen Ausführungsform des erfindungsgemäßen Herstellungsverfahrens kann als Reaktionsprodukt ein Gemisch von mindestens zwei voneinander verschiedenen, Struktureinheiten auf Basis von Glyceriden von 3-Hydroxy- und/oder 3-Alkoxybuttersäuren enthaltenden Lipiden der allgemeinen Formel (III)

R⁶O-CH₂-CH(OR⁷)-CH₂-OR⁸ (III)

wobei in der allgemeinen Formel (III) die Reste R⁶, R⁷ und R⁸, gleich oder verschieden, jeweils unabhängig voneinander
   - einen Rest C₁-C₁₁-Alkyl-C(O)-, insbesondere C₂-C₁₁-Alkyl-C(O)-,
   - einen Rest CH₃-CH(OR⁴)-CH₂-C(O)-, worin der Rest R⁴ die zuvor angegebene Bedeutung hat, insbesondere wobei der Rest R⁴ Wasserstoff oder einen Rest C₁-C₃₀-Alkyl-C(O)-, insbesondere C₁-C₂₁-Alkyl-C(O)-, vorzugsweise C₃-C₂₁-Alkyl-C(O)-, darstellt,
darstellen, jedoch mit der Maßgabe, dass einer der Reste R⁶, R⁷ und R⁸ einen Rest CH₃-CH(OR⁴)-CH₂-C(O)- darstellt,
erhalten werden.

Weiterhin kann gemäß einer anderen besonderen Ausführungsform des erfindungsgemäßen Herstellungsverfahrens als Reaktionsprodukt ein Gemisch von mindestens zwei voneinander verschiedenen, Struktureinheiten auf Basis von Glyceriden von 3-Hydroxy- und/oder 3-Alkoxybuttersäuren enthaltenden Lipiden der allgemeinen Formel (III)

R⁶O-CH₂-CH(OR⁷)-CH₂-OR⁸ (III)

wobei in der allgemeinen Formel (III) die Reste R⁶, R⁷ und R⁸, gleich oder verschieden, jeweils unabhängig voneinander
   - einen Rest C₁₂-C₂₉-Alkyl-C(O)-, insbesondere C₁₉-C₂₉-Alkyl-C(O)-,
   - einen Rest CH₃-CH(OR⁴)-CH₂-C(O)-, worin der Rest R⁴ die zuvor angegebene Bedeutung hat, insbesondere wobei der Rest R⁴ Wasserstoff oder einen Rest C₁-C₃₀-Alkyl-C(O)-, insbesondere C₁-C₂₁-Alkyl-C(O)-, vorzugsweise C₃-C₂₁-Alkyl-C(O)-, darstellt,
darstellen, jedoch mit der Maßgabe, dass einer der Reste R⁶, R⁷ und R⁸ einen Rest CH₃-CH(OR⁴)-CH₂-C(O)- darstellt,
erhalten werden.

Schließlich kann gemäß einer wiederum anderen besonderen Ausführungsform des erfindungsgemäßen Herstellungsverfahrens als Reaktionsprodukt ein Gemisch von mindestens zwei voneinander verschiedenen, Struktureinheiten auf Basis von Glyceriden von 3-Hydroxy- und/oder 3-Alkoxybuttersäuren enthaltenden Lipiden der allgemeinen Formel (III)

R⁶O-CH₂-CH(OR⁷)-CH₂-OR⁸ (III)

wobei in der allgemeinen Formel (III) die Reste R⁶, R⁷ und R⁸, gleich oder verschieden, jeweils unabhängig voneinander
   - einen Rest C₁-C₁₁-Alkyl-C(O)-, insbesondere C₂-C₁₁-Alkyl-C(O)-,
   - einen Rest C₁₂-C₂₉-Alkyl-C(O)-, insbesondere C₁₉-C₂₉-Alkyl-C(O)-,
   - einen Rest CH₃-CH(OR⁴)-CH₂-C(O)-, worin der Rest R⁴ die zuvor angegebene Bedeutung hat, insbesondere wobei der Rest R⁴ Wasserstoff oder einen Rest C₁-C₃₀-Alkyl-C(O)-, insbesondere C₁-C₂₁-Alkyl-C(O)-, vorzugsweise C₃-C₂₁-Alkyl-C(O)-, darstellt,
darstellen, jedoch mit der Maßgabe, dass einer der Reste R⁶, R⁷ und R⁸ einen Rest CH₃-CH(OR⁴)-CH₂-C(O)- darstellt und dass einer der Reste R⁶, R⁷ und R⁸ einen Rest C₁-C₁₁-Alkyl-C(O)-, insbesondere C₂-C₁₁-Alkyl-C(O)-, darstellt und dass einer der Reste R⁶, R⁷ und R⁸ einen Rest C₁₂-C₂₉-Alkyl-C(O)- , insbesondere C₁₉-C₂₉-Alkyl-(O)-, darstellt,
erhalten werden.

Wie zuvor bereits ausgeführt, wird das erfindungsgemäße Verfahren in Abwesenheit von Lösemitteln und/oder ohne jedwedes Lösemittel durchgeführt (d. h. also als Reaktion in Masse bzw. als Reaktion in Substanz bzw. als sogenannte *Bulk Reaction*)*.* Dies hat den Vorteil, dass die erhaltenen Reaktionsprodukte nicht mit Lösemittel verunreinigt sind und kein Lösemittel nach Durchführung des Verfahrens bzw. der Reaktion aufwendig und energieträchtig entfernt und entsorgt bzw. rezykliert werden muss. Überraschenderweise verläuft das Verfahren bzw. die Reaktion dennoch mit hohen Umsätzen und Ausbeuten und zumindest im Wesentlichen ohne signifikante Nebenproduktbildung.

Das erfindungsgemäße Verfahren wird rein beispielhaft und nicht beschränkend durch das folgende allgemeine Reaktionsschema veranschaulicht. Darin bezeichnen der Rest R¹' einen C₁₂-C₂₉-Alkylrest, der Rest R²' einen C₁-C₁₁-Alkylrest und der Rest R³' Wasserstoff oder einen Rest C₁-C₃₀-Alkyl-C(O)-, insbesondere C₁-C₂₁-Alkyl-C(O)-, vorzugsweise C₃-C₂₁-Alkyl-C(O)-. Ein Rest R²'-MCT-Öl bezeichnet ein Triglycerid mit dem Rest R²'. Ein Rest R¹'-MCT-Öl bezeichnet ein Triglycerid mit dem Rest R¹'. 3-Acetoxy-BHB-Anhydrid ist das Anhydrid der 3-Acetoxybuttersäure. 3-R³'-BHB-EE ist der 3-Acetoxybuttersäureethylester. Die Abkürzung "Kat" steht für einen Katalysator (d. h. Enzym). Wie das nachfolgende Reaktionsschema veranschaulicht, lassen sich mit dem erfindungsgemäßen Verfahren unterschiedliche Triglyderide von 3-BHB erhalten, welche weiter randomisiert werden können, insbesondere z. B. mit mittel- und/oder langkettigen Triglyceriden bzw. Fettsäuren, insbesondere mit sogenannten Fischölen.

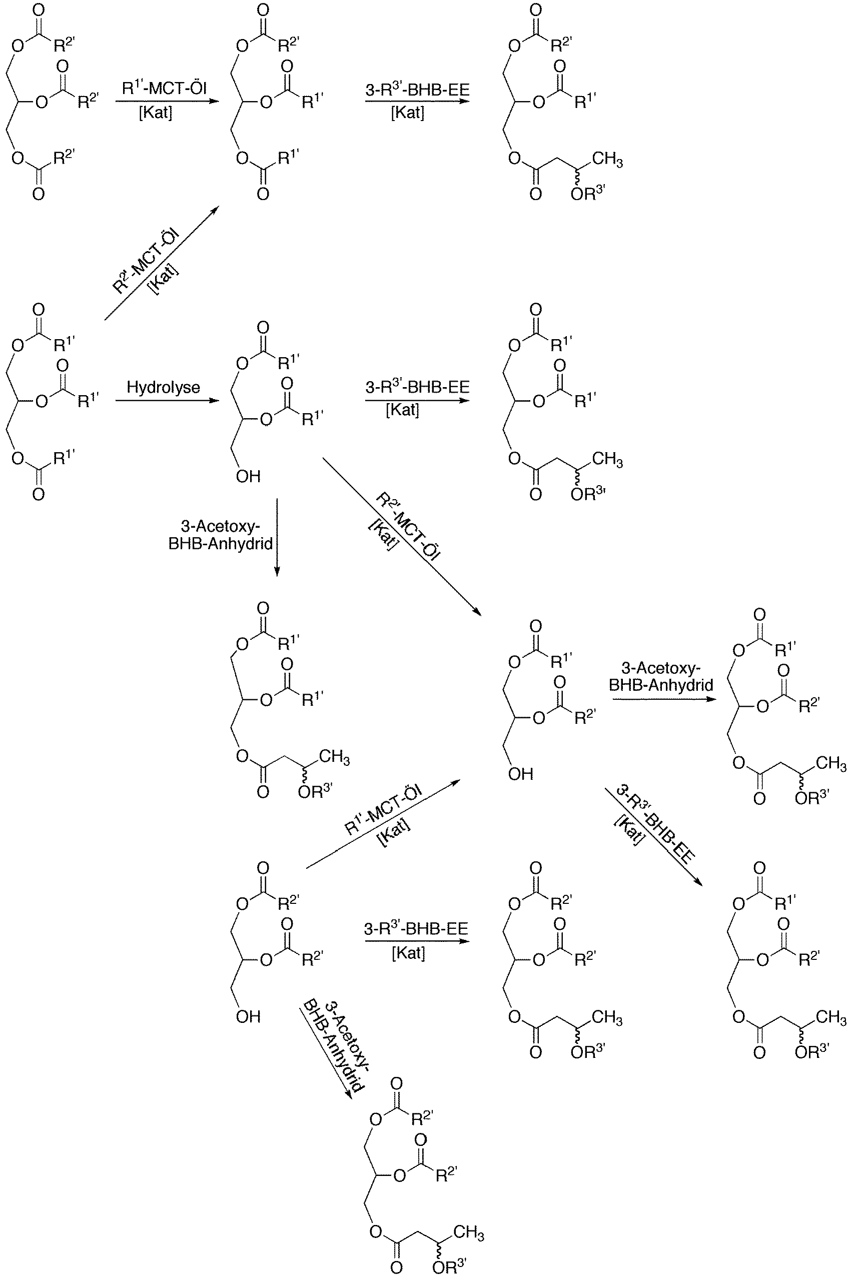

Weiterer Gegenstand - gemäß einem **zweiten** Aspekt der vorliegenden Erfindung - ist das nach dem erfindungsgemäßen Verfahren erhältliche bzw. erfindungsgemäße Reaktionsprodukt (d. h. Gemisch von mindestens zwei voneinander verschiedenen Lipiden, welche Struktureinheiten auf Basis von Glyceriden von 3-Hydroxy- und/oder 3-Alkoxybuttersäuren enthalten) (vgl. Ansprüche 5 bis 14).

Gegenstand gemäß diesem Erfindungsaspekt ist ein Gemisch, umfassend mindestens zwei voneinander verschiedene, Struktureinheiten auf Basis von Glyceriden von 3-Hydroxy- und/oder 3-Alkoxybuttersäuren enthaltende Lipide der allgemeinen Formel (III)

R⁶O-CH₂-CH(OR⁷)-CH₂-OR⁸ (III)

wobei in der allgemeinen Formel (III) die Reste R⁶, R⁷ und R⁸, gleich oder verschieden, jeweils unabhängig voneinander
- einen Rest C₁-C₁₁-Alkyl-C(O)-, insbesondere C₂-C₁₁-Alkyl-C(O)-,
- einen Rest C₁₂-C₂₉-Alkyl-C(O)-, insbesondere C₁₉-C₂₉-Alkyl-C(O)-,
- einen Rest CH₃-CH(OR⁴)-CH₂-C(O)-, worin der Rest R⁴ die zuvor angegebene Bedeutung hat, insbesondere wobei der Rest R⁴ Wasserstoff oder einen Rest C₁-C₃₀-Alkyl-C(O)-, insbesondere C₁-C₂₁-Alkyl-C(O)-, vorzugsweise C₃-C₂₁-Alkyl-C(O)-, darstellt,
darstellen, jedoch mit der Maßgabe, dass ein oder zwei der Reste R⁶, R⁷ und R⁸ einen Rest CH₃-CH(OR⁴)-CH₂-C(O)- darstellt.

Insbesondere ist Gegenstand der vorliegenden Erfindung gemäß diesem Erfindungsaspekt ein Gemisch von mindestens zwei voneinander verschiedenen Lipiden, welche Struktureinheiten auf Basis von Glyceriden von 3-Hydroxy- und/oder 3-Alkoxybuttersäuren enthalten, wie in den Ansprüchen 5 bis 14 definiert.

Insbesondere ist Gegenstand der vorliegenden Erfindung gemäß diesem Erfindungsaspekt ein Gemisch, umfassend mindestens zwei voneinander verschiedene, Struktureinheiten auf Basis von Glyceriden von 3-Hydroxy- und/oder 3-Alkoxybuttersäuren enthaltende Lipide,wobei die Struktureinheiten auf Basis von Glyceriden von 3-Hydroxy- und/oder 3-Alkoxybuttersäuren enthaltenden Lipide der allgemeinen Formel (III)

R⁶O-CH₂-CH(OR⁷)-CH₂-OR⁸ (III)

wobei in der allgemeinen Formel (III) die Reste R⁶, R⁷ und R⁸, gleich oder verschieden, jeweils unabhängig voneinander
   - einen Rest C₁-C₁₁-Alkyl-C(O)-, insbesondere C₂-C₁₁-Alkyl-C(O)-,
   - einen Rest C₁₂-C₂₉-Alkyl-C(O)-, insbesondere C₁₉-C₂₉-Alkyl-C(O)-,
   - einen Rest CH₃-CH(OR⁴)-CH₂-C(O)-, worin der Rest R⁴ die zuvor angegebene Bedeutung hat, insbesondere wobei der Rest R⁴ Wasserstoff oder einen Rest C₁-C₃₀-Alkyl-C(O)-, insbesondere C₁-C₂₁-Alkyl-C(O)-, vorzugsweise C₃-C₂₁-Alkyl-C(O)-, darstellt,
darstellen, jedoch mit der Maßgabe, dass einer oder zwei der Reste R⁶, R⁷ und R⁸ einen Rest CH₃-CH(OR⁴)-CH₂-C(O)- darstellt (und vorzugsweise mit der weiteren Maßgabe dass für den Fall, dass alle Reste R⁶, R⁷ und R⁸ einen Rest CH₃-CH(OR⁴)-CH₂-C(O)- darstellen, bei mindestens einem der Reste R⁶, R⁷ und R⁸ im Rest CH₃-CH(OR⁴)-CH₂-C(O)- der Rest R⁴ keinen Wasserstoff darstellt),
entsprechen.

Weiterhin ist Gegenstand der vorliegenden Erfindung gemäß diesem Erfindungsaspekt ein Gemisch, umfassend mindestens zwei voneinander verschiedene, Struktureinheiten auf Basis von Glyceriden von 3-Hydroxy- und/oder 3-Alkoxybuttersäuren enthaltende Lipide, insbesondere wie zuvor definiert,
wobei die Struktureinheiten auf Basis von Glyceriden von 3-Hydroxy- und/oder 3-Alkoxybuttersäuren enthaltenden Lipide der allgemeinen Formel (III)

   R⁶O-CH₂-CH(OR⁷)-CH₂-OR⁸ (III)

   wobei in der allgemeinen Formel (III) die Reste R⁶, R⁷ und R⁸, gleich oder verschieden, jeweils unabhängig voneinander
      - einen Rest vom Typ lineares (geradkettiges) oder verzweigtes, gesättigtes oder ein- oder mehrfach ungesättigtes C₁-C₁₁-Alkyl-C(O)-, insbesondere lineares (geradkettiges) oder verzweigtes, gesättigtes oder ein- oder mehrfach ungesättigtes C₂-C₁₁-Alkyl-C(O)-,
      - einen Rest vom Typ lineares (geradkettiges) oder verzweigtes, gesättigtes oder ein- oder mehrfach ungesättigtes C₁₂-C₂₉-Alkyl-C(O)-, insbesondere lineares (geradkettiges) oder verzweigtes, gesättigtes oder ein- oder mehrfach ungesättigtes C₁₉-C₂₉-Alkyl-C(O)-,
      - einen Rest vom Typ lineares (geradkettiges) oder verzweigtes, gesättigtes oder ein- oder mehrfach ungesättigtes CH₃-CH(OR⁴)-CH₂-C(O)-, worin der Rest R⁴ die zuvor angegebene Bedeutung hat, insbesondere wobei der Rest R⁴ Wasserstoff oder einen Rest C₁-C₃₀-Alkyl-C(O)-, insbesondere C₁-C₂₁-Alkyl-C(O)-, vorzugsweise C₃-C₂₁-Alkyl-C(O)-, darstellt,
   darstellen, jedoch mit der Maßgabe, dass mindestens einer der Reste R⁶, R⁷ und R⁸ einen Rest CH₃-CH(OR⁴)-CH₂-C(O)- darstellt.
entsprechen.

Wiederum weiterer Gegenstand der vorliegenden Erfindung gemäß diesem Erfindungsaspekt ist ein Gemisch, umfassend mindestens zwei voneinander verschiedene, Struktureinheiten auf Basis von Glyceriden von 3-Hydroxy- und/oder 3-Alkoxybuttersäuren enthaltende Lipide, insbesondere wie zuvor definiert,
wobei die Struktureinheiten auf Basis von Glyceriden von 3-Hydroxy- und/oder 3-Alkoxybuttersäuren enthaltenden Lipide der allgemeinen Formel (III)

   R⁶O-CH₂-CH(OR⁷)-CH₂-OR⁸ (III)

   wobei in der allgemeinen Formel (III) die Reste R⁶, R⁷ und R⁸, gleich oder verschieden, jeweils unabhängig voneinander
      - einen Rest C₁-C₁₁-Alkyl-C(O)-, insbesondere C₂-C₁₁-Alkyl-C(O)-,
      - einen Rest C₁₂-C₂₉-Alkyl-C(O)-, insbesondere C₁₉-C₂₉-Alkyl-C(O)-,
      - einen Rest CH₃-CH(OR⁴)-CH₂-C(O)-, worin der Rest R⁴ die zuvor angegebene Bedeutung hat, insbesondere wobei der Rest R⁴ Wasserstoff oder einen Rest C₁-C₃₀-Alkyl-C(O)-, insbesondere C₁-C₂₁-Alkyl-C(O)-, vorzugsweise C₃-C₂₁-Alkyl-C(O)-, darstellt,
   darstellen, jedoch mit der Maßgabe, dass zwei Reste R⁶, R⁷ und R⁸ einen Rest CH₃-CH(OR⁴)-CH₂-C(O)- darstellen,
entsprechen.

Ein ebenfalls weiterer Gegenstand der vorliegenden Erfindung gemäß diesem Erfindungsaspekt ist ein Gemisch, umfassend mindestens zwei voneinander verschiedene, Struktureinheiten auf Basis von Glyceriden von 3-Hydroxy- und/oder 3-Alkoxybuttersäuren enthaltende Lipide, insbesondere wie zuvor definiert,
wobei die Struktureinheiten auf Basis von Glyceriden von 3-Hydroxy- und/oder 3-Alkoxybuttersäuren enthaltenden Lipide der allgemeinen Formel (III)

   R⁶O-CH₂-CH(OR⁷)-CH₂-OR⁸ (III)

   wobei in der allgemeinen Formel (III) die Reste R⁶, R⁷ und R⁸, gleich oder verschieden, jeweils unabhängig voneinander
      - einen Rest C₁-C₁₁-Alkyl-C(O)-, insbesondere C₂-C₁₁-Alkyl-C(O)-,
      - einen Rest CH₃-CH(OR⁴)-CH₂-C(O)-, worin der Rest R⁴ die zuvor angegebene Bedeutung hat, insbesondere wobei der Rest R⁴ Wasserstoff oder einen Rest C₁-C₃₀-Alkyl-C(O)-, insbesondere C₁-C₂₁-Alkyl-C(O)-, vorzugsweise C₃-C₂₁-Alkyl-C(O)-, darstellt,
   darstellen, jedoch mit der Maßgabe, dass zwei Reste R⁶, R⁷ und R⁸ einen Rest CH₃-CH(OR⁴)-CH₂-C(O)- darstellen,
entsprechen.

Darüber hinaus ist Gegenstand der vorliegenden Erfindung gemäß diesem Erfindungsaspekt auch ein Gemisch, umfassend mindestens zwei voneinander verschiedene, Struktureinheiten auf Basis von Glyceriden von 3-Hydroxy- und/oder 3-Alkoxybuttersäuren enthaltende Lipide, insbesondere wie zuvor definiert,
wobei die Struktureinheiten auf Basis von Glyceriden von 3-Hydroxy- und/oder 3-Alkoxybuttersäuren enthaltenden Lipide der allgemeinen Formel (III)

   R⁶O-CH₂-CH(OR⁷)-CH₂-OR⁸ (III)

   wobei in der allgemeinen Formel (III) die Reste R⁶, R⁷ und R⁸, gleich oder verschieden, jeweils unabhängig voneinander
      - einen Rest C₁₂-C₂₉-Alkyl-C(O)-, insbesondere C₁₉-C₂₉-Alkyl-C(O)-,
      - einen Rest CH₃-CH(OR⁴)-CH₂-C(O)-, worin der Rest R⁴ die zuvor angegebene Bedeutung hat, insbesondere wobei der Rest R⁴ Wasserstoff oder einen Rest C₁-C₃₀-Alkyl-C(O)-, insbesondere C₁-C₂₁-Alkyl-C(O)-, vorzugsweise C₃-C₂₁-Alkyl-C(O)-, darstellt,
   darstellen, jedoch mit der Maßgabe, dass zwei Reste R⁶, R⁷ und R⁸ einen Rest CH₃-CH(OR⁴)-CH₂-C(O)- darstellen,
entsprechen.

Weiterer Gegenstand der vorliegenden Erfindung gemäß diesem Erfindungsaspekt ist ein Gemisch, umfassend mindestens zwei voneinander verschiedene, Struktureinheiten auf Basis von Glyceriden von 3-Hydroxy- und/oder 3-Alkoxybuttersäuren enthaltende Lipide, insbesondere wie zuvor definiert,
wobei die Struktureinheiten auf Basis von Glyceriden von 3-Hydroxy- und/oder 3-Alkoxybuttersäuren enthaltenden Lipide der allgemeinen Formel (III)

   R⁶O-CH₂-CH(OR⁷)-CH₂-OR⁸ (III)

   wobei in der allgemeinen Formel (III) die Reste R⁶, R⁷ und R⁸, gleich oder verschieden, jeweils unabhängig voneinander
      - einen Rest C₁-C₁₁-Alkyl-C(O)-, insbesondere C₂-C₁₁-Alkyl-C(O)-,
      - einen Rest C₁₂-C₂₉-Alkyl-C(O)-, insbesondere C₁₉-C₂₉-Alkyl-C(O)-,
      - einen Rest CH₃-CH(OR⁴)-CH₂-C(O)-, worin der Rest R⁴ die zuvor angegebene Bedeutung hat, insbesondere wobei der Rest R⁴ Wasserstoff oder einen Rest C₁-C₃₀-Alkyl-C(O)-, insbesondere C₁-C₂₁-Alkyl-C(O)-, vorzugsweise C₃-C₂₁-Alkyl-C(O)-, darstellt,
   darstellen, jedoch mit der Maßgabe, dass einer der Reste R⁶, R⁷ und R⁸ einen Rest CH₃-CH(OR⁴)-CH₂-C(O)- darstellt,
entsprechen.

Weiterhin betrifft die vorliegende Erfindung gemäß diesem Erfindungsaspekt ein Gemisch, umfassend mindestens zwei voneinander verschiedene, Struktureinheiten auf Basis von Glyceriden von 3-Hydroxy- und/oder 3-Alkoxybuttersäuren enthaltende Lipide, insbesondere wie zuvor definiert,
wobei die Struktureinheiten auf Basis von Glyceriden von 3-Hydroxy- und/oder 3-Alkoxybuttersäuren enthaltenden Lipide der allgemeinen Formel (III)

   R⁶O-CH₂-CH(OR⁷)-CH₂-OR⁸ (III)

   wobei in der allgemeinen Formel (III) die Reste R⁶, R⁷ und R⁸, gleich oder verschieden, jeweils unabhängig voneinander
      - einen Rest C₁-C₁₁-Alkyl-C(O)-, insbesondere C₂-C₁₁-Alkyl-C(O)-,
      - einen Rest CH₃-CH(OR⁴)-CH₂-C(O)-, worin der Rest R⁴ die zuvor angegebene Bedeutung hat, insbesondere wobei der Rest R⁴ Wasserstoff oder einen Rest C₁-C₃₀-Alkyl-C(O)-, insbesondere C₁-C₂₁-Alkyl-C(O)-, vorzugsweise C₃-C₂₁-Alkyl-C(O)-, darstellt,
   darstellen, jedoch mit der Maßgabe, dass einer der Reste R⁶, R⁷ und R⁸ einen Rest CH₃-CH(OR⁴)-CH₂-C(O)- darstellt,
entsprechen.

Auch betrifft die vorliegende Erfindung gemäß diesem Erfindungsaspekt ein Gemisch, umfassend mindestens zwei voneinander verschiedene, Struktureinheiten auf Basis von Glyceriden von 3-Hydroxy- und/oder 3-Alkoxybuttersäuren enthaltende Lipide, insbesondere wie zuvor definiert,
wobei die Struktureinheiten auf Basis von Glyceriden von 3-Hydroxy- und/oder 3-Alkoxybuttersäuren enthaltenden Lipide der allgemeinen Formel (III)

   R⁶O-CH₂-CH(OR⁷)-CH₂-OR⁸ (III)

   wobei in der allgemeinen Formel (III) die Reste R⁶, R⁷ und R⁸, gleich oder verschieden, jeweils unabhängig voneinander
      - einen Rest C₁₂-C₂₉-Alkyl-C(O)-, insbesondere C₁₉-C₂₉-Alkyl-C(O)-,
      - einen Rest CH₃-CH(OR⁴)-CH₂-C(O)-, worin der Rest R⁴ die zuvor angegebene Bedeutung hat, insbesondere wobei der Rest R⁴ Wasserstoff oder einen Rest C₁-C₃₀-Alkyl-C(O)-, insbesondere C₁-C₂₁-Alkyl-C(O)-, vorzugsweise C₃-C₂₁-Alkyl-C(O)-, darstellt,
   darstellen, jedoch mit der Maßgabe, dass einer der Reste R⁶, R⁷ und R⁸ einen Rest CH₃-CH(OR⁴)-CH₂-C(O)- darstellt,
entsprechen.

Schließlich ist wiederum weiterer Gegenstand der vorliegenden Erfindung gemäß diesem Erfindungsaspekt ein Gemisch, umfassend mindestens zwei voneinander verschiedene, Struktureinheiten auf Basis von Glyceriden von 3-Hydroxy- und/oder 3-Alkoxybuttersäuren enthaltende Lipide, insbesondere wie zuvor definiert,
wobei die Struktureinheiten auf Basis von Glyceriden von 3-Hydroxy- und/oder 3-Alkoxybuttersäuren enthaltenden Lipide der allgemeinen Formel (III)

   R⁶O-CH₂-CH(OR⁷)-CH₂-OR⁸ (III)

   wobei in der allgemeinen Formel (III) die Reste R⁶, R⁷ und R⁸, gleich oder verschieden, jeweils unabhängig voneinander
      - einen Rest C₁-C₁₁-Alkyl-C(O)-, insbesondere C₂-C₁₁-Alkyl-C(O)-,
      - einen Rest C₁₂-C₂₉-Alkyl-C(O)-, insbesondere C₁₉-C₂₉-Alkyl-C(O)-,
      - einen Rest CH₃-CH(OR⁴)-CH₂-C(O)-, worin der Rest R⁴ die zuvor angegebene Bedeutung hat, insbesondere wobei der Rest R⁴ Wasserstoff oder einen Rest C₁-C₃₀-Alkyl-C(O)-, insbesondere C₁-C₂₁-Alkyl-C(O)-, vorzugsweise C₃-C₂₁-Alkyl-C(O)-, darstellt,
   darstellen, jedoch mit der Maßgabe, dass einer der Reste R⁶, R⁷ und R⁸ einen Rest CH₃-CH(OR⁴)-CH₂-C(O)- darstellt und dass einer der Reste R⁶, R⁷ und R⁸ einen Rest C₁-C₁₁-Alkyl-C(O)-, insbesondere C₂-C₁₁-Alkyl-C(O)-, darstellt und dass einer der Reste R⁶, R⁷ und R⁸ einen Rest C₁₂-C₂₉-Alkyl-C(O)- , insbesondere C₁₉-C₂₉-Alkyl-C(O)-, darstellt,
   entsprechen.

Gegenstand der vorliegenden Erfindung gemäß diesem Erfindungsaspekt ist also ein wie zuvor definiertes Gemisch, welches mindestens zwei voneinander verschiedene Lipide, welche Struktureinheiten auf Basis von Glyceriden von 3-Hydroxy- und/oder 3-Alkoxybuttersäuren enthalten, wie zuvor definiert, umfasst.

Insbesondere ist wiederum weiterer Gegenstand der vorliegenden Erfindung gemäß diesem Erfindungsaspekt ein wie zuvor definiertes Gemisch, welches mindestens drei voneinander verschiedene Lipide, welche Struktureinheiten auf Basis von Glyceriden von 3-Hydroxy- und/oder 3-Alkoxybuttersäuren enthalten, wie zuvor definiert, umfasst.

Das nach dem erfindungsgemäßen Verfahren erhältliche bzw. erfindungsgemäße Reaktionsprodukt, wie zuvor definiert, und/oder das nach dem erfindungsgemäßen Herstellungsverfahren erhältliche bzw. erfindungsgemäße Gemisch, wie zuvor definiert, weist bzw. weisen gegenüber dem Stand der Technik eine Vielzahl von Vorteilen und Besonderheiten auf:
Wie die Anmelderin überraschend herausgefunden hat, eignet sich das nach dem erfindungsgemäßen Verfahren erhältliche bzw. erfindungsgemäße Reaktionsprodukt, wie zuvor definiert, und/oder das nach dem erfindungsgemäßen Herstellungsverfahren erhältliche bzw. erfindungsgemäße Gemisch, wie zuvor definiert, insbesondere als Präkursor bzw. Metabolit von 3-Hydroxybuttersäure bzw. deren Salzen, da dieses bzw. diese einerseits physiologisch, insbesondere im Magen/Darm-Trakt, zu 3-Hydroxybuttersäure bzw. deren Salzen umgesetzt wird/werden und andererseits gleichzeitig eine gute physiologische Kompatibilität bzw. Verträglichkeit aufweist/aufweisen, insbesondere im Hinblick auf Nichttoxizität und akzeptable organoleptische Eigenschaften.

Darüber hinaus ist/sind das nach dem erfindungsgemäßen Verfahren erhältliche bzw. erfindungsgemäße Reaktionsprodukt, wie zuvor definiert, und/oder das nach dem erfindungsgemäßen Herstellungsverfahren erhältliche bzw. erfindungsgemäße Gemisch, wie zuvor definiert, ohne Weiteres auf synthetischem Wege auch in großtechnischem Maßstab zugänglich bzw. verfügbar, und zwar auch mit der erforderlichen pharmazeutischen bzw. pharmakologischen Qualität.

Zudem kann/können das nach dem erfindungsgemäßen Verfahren erhältliche bzw. erfindungsgemäße Reaktionsprodukt, wie zuvor definiert, und/oder das nach dem erfindungsgemäßen Herstellungsverfahren erhältliche bzw. erfindungsgemäße Gemisch, wie zuvor definiert, erforderlichenfalls in enantiomerenreiner bzw. enantiomerenangereicherter Form bereitgestellt werden.

Das nach dem erfindungsgemäßen Verfahren erhältliche bzw. erfindungsgemäße Reaktionsprodukt, wie zuvor definiert, und/oder das nach dem erfindungsgemäßen Herstellungsverfahren erhältliche bzw. erfindungsgemäße Gemisch, wie zuvor definiert, stellt/stellen somit ein effizientes pharmakologisches Wirkstoff-Target im Rahmen einer Ketokörper-Therapie des menschlichen oder tierischen Körpers dar.

Nachfolgend werden noch die übrigen Erfindungsaspekte weiterführende erläutert und im Detail beschrieben.

Gegenstand der vorliegenden Erfindung - gemäß einem **dritten** Aspekt der vorliegenden Erfindung - ist eine pharmazeutische Zusammensetzung, insbesondere ein Arzneimittel oder Medikament, welche(s) ein nach dem erfindungsgemäßen Herstellungsverfahren erhältliches bzw. erfindungsgemäßes Reaktionsprodukt, wie zuvor definiert, und/oder ein nach dem erfindungsgemäßen Herstellungsverfahren erhältliches bzw. erfindungsgemäßes Gemisch, wie zuvor definiert, umfasst.

Insbesondere betrifft die vorliegende Erfindung gemäß diesem Erfindungsaspekt eine pharmazeutische Zusammensetzung zur prophylaktischen und/oder therapeutischen Behandlung bzw. zur Verwendung bei der prophylaktischen und/oder therapeutischen Behandlung von Erkrankungen des menschlichen oder tierischen Körpers. Hierbei kann es sich insbesondere um Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien, handeln.

Wiederum weiterer Gegenstand der vorliegenden Erfindung - gemäß einem **vierten** Aspekt der vorliegenden Erfindung - ist ein nach dem erfindungsgemäßen Herstellungsverfahren erhältliches bzw. erfindungsgemäßes Reaktionsprodukt, wie zuvor definiert, und/oder ein nach dem erfindungsgemäßen Herstellungsverfahren erhältliches bzw. erfindungsgemäßes Gemisch, wie zuvor definiert, zur prophylaktischen und/oder therapeutischen Behandlung bzw. zur Verwendung bei der prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

Gleichermaßen weiterer Gegenstand der vorliegenden Erfindung - gemäß einem **fünften** Aspekt der vorliegenden Erfindung - ist die Verwendung eines nach dem erfindungsgemäßen Herstellungsverfahren erhältlichen bzw. erfindungsgemäßen Reaktionsprodukts, wie zuvor definiert, und/oder eines nach dem erfindungsgemäßen Herstellungsverfahren erhältlichen bzw. erfindungsgemäßen Gemischs, wie zuvor definiert, zur prophylaktischen und/oder therapeutischen Behandlung bzw. zur Herstellung eines Arzneimittels zur prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

Gleichermaßen eignet sich das nach dem erfindungsgemäßen Herstellungsverfahren erhältliche und/oder erfindungsgemäße Reaktionsprodukt, wie zuvor definiert, und/oder das nach dem erfindungsgemäßen Herstellungsverfahren erhältliche bzw. erfindungsgemäße Gemisch, wie zuvor definiert, für die Verwendung zur prophylaktischen und/oder therapeutischen Behandlung bzw. zur Herstellung eines Arzneimittels zur prophylaktischen und/oder therapeutischen Behandlung bzw. zur Anwendung von/bei katabolen Stoffwechsellagen, wie Hunger, Diäten oder kohlenhydratarmer Ernährung.

Gleichermaßen weiterer Gegenstand der vorliegenden Erfindung - gemäß einem **sechsten** Aspekt der vorliegenden Erfindung - ist ein Nahrungsmittel- und/oder Lebensmittelerzeugnis, welches ein nach dem erfindungsgemäßen Herstellungsverfahren erhältliches bzw. erfindungsgemäßes Reaktionsprodukt, wie zuvor definiert, und/oder ein nach dem erfindungsgemäßen Herstellungsverfahren erhältliches bzw. erfindungsgemäßes Gemisch, wie zuvor definiert, umfasst.

Gemäß einer besonderen Ausführungsform kann das Nahrungsmittel- und/oder Lebensmittelerzeugnis insbesondere ein Nahrungsergänzungsmittel, ein funktionelles Lebensmittel (*Functional Food*)*,* ein *Novel Food,* ein Lebensmittelzusatzstoff, ein Nahrungszusatz, ein diätetisches Lebensmittel, ein Power-Snack, ein Appetitzügler oder ein Kraft- und/oder Ausdauersport-Supplement sein.

Schließlich ist wiederum weiterer Gegenstand der vorliegenden Erfindung - gemäß einem **siebten** Aspekt der vorliegenden Erfindung - die Verwendung eines nach dem erfindungsgemäßen Herstellungsverfahren erhältlichen bzw. erfindungsgemäßen Reaktionsprodukts, wie zuvor definiert, und/oder eines nach dem erfindungsgemäßen Herstellungsverfahren erhältlichen bzw. erfindungsgemäßen Gemischs, wie zuvor definiert, in einem Nahrungsmittel- und/oder Lebensmittelerzeugnis.

Gemäß diesem Erfindungsaspekt kann das Nahrungsmittel- und/oder Lebensmittelerzeugnis insbesondere ein Nahrungsergänzungsmittel, ein funktionelles Lebensmittel (*Functional Food*)*,* ein *Novel Food,* ein Lebensmittelzusatzstoff, ein Nahrungszusatz, ein diätetisches Lebensmittel, ein Power-Snack, ein Appetitzügler oder ein Kraft- und/oder Ausdauersport-Supplement sein.

Weitere Ausgestaltungen, Abwandlungen und Variationen der vorliegenden Erfindung sind für den Fachmann beim Lesen der Beschreibung ohne Weiteres erkennbar oder realisierbar, ohne dass er dabei den Rahmen der vorliegenden Erfindung verlässt.

Die vorliegende Erfindung wird anhand der nachfolgenden Ausführungsbeispiele veranschaulicht, welche die vorliegende Erfindung jedoch keinesfalls beschränken sollen, sondern lediglich die beispielhafte und nichtlimitierende Durchführungsweise und Ausgestaltung der vorliegenden Erfindung erläutern sollen.

### AUSFÜHRUNGSBEISPIELE:

### Herstellungsbeispiele

Das erfindungsgemäße Herstellungsverfahren wird anhand der nachfolgenden Ausführungsbeispiele veranschaulicht. Die diesbezüglichen Reaktionsschemata sind im allgemeinen Beschreibungsteil dargestellt und erläutert.

### Beispiel 1:

### Herstellung von gemischten Triglyceriden mit Ketten auf Basis von Long Chain Fatty Acid (LCFA), Medium Chain Fatty Acid (MCFA) und 3-Hydroxybuttersäure (BHB bzw. 3-BHB) (d. h. gemischte Long Chain Fatty Acid (LCFA), Medium Chain Fatty Acid (MCFA) und 3-Hydroxybuttersäure(3-BHB)-Triglyceride)

In einem 2.000-ml-Mehrhalskolben mit Dephlegmator (Partialkondensator) und Destillationsbrücke werden 500 g eines mittelkettigen Triglycerids (C₈ / C₁₀ Fettsäure enthaltendes Triglycerid, Verhältnis 60 % / 40 %) und 900 g eines langkettigen, raffinierten, gebleichten und desodorisierten Fischöls (EPA / DHA-Gehalt 20 bis 50 %, Verhältnis 50 : 50) unter Rühren bei 50 bis 70 °C und unter Vakuum (< 100 mbar) für 12 bis 24 h mittels 14 g immobilisiertem Enzym (CALB) zur Reaktion gebracht. Danach wird das Enzym abfiltriert. Das Reaktionsprodukt ist ein randomisiertes LCF / MCF-Triglycerid.

In einem 2.000-ml-Mehrhalskolben mit Dephlegmator (Partialkondensator) und Destillationsbrücke werden 1.000 g des oben hergestellten, randomisierten LCF / MCF-Triglycerids mit 500 g (R)/(S)-3-Hydroxybuttersäureethylester (3-BHB-Ethylester) und 15 g immobilisiertem Enzym (CALB, z. B. immobilisiertes Enzym, wie z. B. CALB-Lipase auf Polymerträger, abgeleitet von *Candida antarctica,* z. B. Novozym^{®} 435 von der Fa. Sigma-Aldrich bzw. Merck oder Lipozym^{®} 435 von der Fa. Strem Chemicals, Inc.) bei 50 bis 70 °C und unter Vakuum (< 500 mbar) für 24 bis 36 h umgeestert. Danach wird das Enzym abfiltriert.

Das Reaktionsprodukt ist ein LCF / MCF / 3-BHB-Triglycerid bzw. ein strukturiertes Lipid.

Spaltungsversuche (Spaltversuche) dieses Reaktionsprodukts in einem Magen- oder Darmmedium (FaSSGF-Medium, welches den Magen simuliert, oder FaSSIF-Medium, welches den Darmtrakt simuliert), jeweils in Anwesenheit oder in Abwesenheit von Pankreatin, belegen die Spaltung zu 3-BHB in freier Form. Diese Spaltungsversuche belegen, dass erfindungsgemäße Struktureinheiten auf Basis von Glyceriden von 3-Hydroxy- und/oder 3-Alkoxybuttersäuren enthaltende Lipide (hier konkret das erhaltene Reaktionsprodukt) effiziente Präkursoren bzw. Metabolite der freien 3-Hydroxybuttersäure bzw. deren Salze darstellen, insbesondere im Hinblick auf ihre beabsichtigte Wirkung, welche zudem in physiologisch verträglicher bzw. physiologisch kompatibler Form vorliegen.

### Beispiel 2:

### Herstellung von gemischten Triglyceriden mit Ketten auf Basis von Long Chain Fatty Acid (LCFA) und 3-Hydroxybuttersäure (BHB bzw. 3-BHB) (d. h. gemischte Long Chain Fatty Acid (LCFA) und 3-Hydroxybuttersäure(3-BHB)-Triglyceride)

In einem 1.000-ml-Mehrhalskolben mit Dephlegmator (Partialkondensator) und Destillationsbrücke werden 500 g eines langkettigen, raffinierten, gebleichten und desodorisierten Fischöls (EPA / DHA Gehalt 20 bis 50 %, Verhältnis 50 : 50) mit 200 g (R)/(S)-3-Hydroxybuttersäureethylester (3-BHB-Ethylester) und 15 g immobilisiertem Enzym (CALB) bei 50 bis 70 °C und unter Vakuum (< 500 mbar) für 24 bis 36 h umgeestert. Danach wird das Enzym abfiltriert.

Das Reaktionsprodukt ist ein LCF / 3-BHB-Triglycerid bzw. ein strukturiertes Lipid.

Spaltungsversuche (Spaltversuche) dieses Reaktionsprodukts in einem Magen- oder Darmmedium (FaSSGF-Medium, welches den Magen simuliert, oder FaSSIF-Medium, welches den Darmtrakt simuliert), jeweils in Anwesenheit oder in Abwesenheit von Pankreatin, belegen die Spaltung zu 3-BHB in freier Form. Diese Spaltungsversuche belegen, dass erfindungsgemäße Struktureinheiten auf Basis von Glyceriden von 3-Hydroxy- und/oder 3-Alkoxybuttersäuren enthaltende Lipide (hier konkret das erhaltene Reaktionsprodukt) effiziente Präkursoren bzw. Metabolite der freien 3-Hydroxybuttersäure bzw. deren Salze darstellen, insbesondere im Hinblick auf ihre beabsichtigte Wirkung, welche zudem in physiologisch verträglicher bzw. physiologisch kompatibler Form vorliegen.

### Beispiel 3:

### Herstellung von gemischten Medium Chain Fatty Acid (MCFA) / 3-Hydroxybuttersäure(3-BHB)-Triglyceriden

In einem 1.000-ml-Mehrhalskolben mit Dephlegmator (Partialkondensator) und Destillationsbrücke werden 500 g mittelkettiges Triglycerid (C₈ / C₁₀-Fettsäure enthaltendes Triglycerid, Verhältnis 60 % / 40 %) mit 120 g (R)/(S)-3-Hydroxybuttersäureethylester (3-BHB-Ethylester) und 15 g immobilisiertem Enzym (CALB) bei 50 bis 70 °C und unter Vakuum (< 500 mbar) für 24 bis 36 h umgeestert. Danach wird das Enzym abfiltriert.

Das Reaktionsprodukt ist ein MCF / 3-BHB-Triglycerid bzw. ein strukturiertes Lipid.

Spaltungsversuche (Spaltversuche) dieses Reaktionsprodukts in einem Magen- oder Darmmedium (FaSSGF-Medium, welches den Magen simuliert, oder FaSSIF-Medium, welches den Darmtrakt simuliert), jeweils in Anwesenheit oder in Abwesenheit von Pankreatin, belegen die Spaltung zu 3-BHB in freier Form. Diese Spaltungsversuche belegen, dass erfindungsgemäße Struktureinheiten auf Basis von Glyceriden von 3-Hydroxy- und/oder 3-Alkoxybuttersäuren enthaltende Lipide (hier konkret das erhaltene Reaktionsprodukt) effiziente Präkursoren bzw. Metabolite der freien 3-Hydroxybuttersäure bzw. deren Salze darstellen, insbesondere im Hinblick auf ihre beabsichtigte Wirkung, welche zudem in physiologisch verträglicher bzw. physiologisch kompatibler Form vorliegen.

### Beispiel 4:

### Herstellung von gemischten Medium Chain Fatty Acid (MCFA) und 3-Acetoxybuttersäure(Ac-BHB)-Triglyceriden aus Partialglyceriden und 3-Acetoxybuttersäure-Anhydriden

In einem 1.000-ml-Mehrhalskolben mit Dephlegmator (Partialkondensator) und Destillationsbrücke werden 25 g einer (R)/(S)-3-Hydroxybuttersäure (3-BHB) in 95 g Essigsäure vorgelegt. Zu der Reaktionsmischung werden bei 80 °C unter N₂-Atmosphäre 90 g Essigsäureanhydrid innerhalb von einer Stunde zugetropft. Das Reaktionsgemisch wird bei 80 °C für weitere 4 bis 5 h gerührt. Zu der Reaktionsmischung werden bei 80 °C 3 g eines mittelkettigen Mono-/Di-Glycerids (C₈ / C₁₀-Fettsäure im Verhältnis 60 %/40 %; Glyceridverteilung: Monoester 30 bis 70 %, Di-Ester 10 bis 30 %, Tri-Ester 1 bis 5 %) hinzugegeben und für 4 bis 5 h gerührt. Die entstehende 3-Acetoxybuttersäure bzw. Essigsäure wird im Vakuum (< 50 mbar) bei 100 bis 120 °C abdestilliert.

Das Reaktionsprodukt ist ein MCF / 3-Acetoxy-BHB-Triglycerid.

Spaltungsversuche (Spaltversuche) dieses Reaktionsprodukts in einem Magen- oder Darmmedium (FaSSGF-Medium, welches den Magen simuliert, oder FaSSIF-Medium, welches den Darmtrakt simuliert), jeweils in Anwesenheit oder in Abwesenheit von Pankreatin, belegen die Spaltung zu 3-BHB in freier Form. Diese Spaltungsversuche belegen, dass erfindungsgemäße Struktureinheiten auf Basis von Glyceriden von 3-Hydroxy- und/oder 3-Alkoxybuttersäuren enthaltende Lipide (hier konkret das erhaltene Reaktionsprodukt) effiziente Präkursoren bzw. Metabolite der freien 3-Hydroxybuttersäure bzw. deren Salze darstellen, insbesondere im Hinblick auf ihre beabsichtigte Wirkung, welche zudem in physiologisch verträglicher bzw. physiologisch kompatibler Form vorliegen.

### Beispiel 5:

### Herstellung von gemischten Long Chain Fatty Acid (LCFA) und 3-Acetoxybuttersäure(Ac-BHB)-Triglyceriden aus Partialglyceriden und 3-Acetoxybuttersäure-Anhydriden

In einem 1.000-ml-Mehrhalskolben mit Dephlegmator (Partialkondensator) und Destillationsbrücke werden 500 g eines langkettigen, raffinierten, gebleichten und desodorisierten Fischöls (EPA / DHA Gehalt 20 bis 50 %, Verhältnis 50 : 50) mit 250 g Wasser 0,4 g Enzym Lipase bei 40 °C unter Rühren und N₂-Atmosphäre 8 bis 12 h selektiv gespalten. Es werden hauptsächlich Fettsäuren < C₂₀ abgespalten. Danach werden die Phasen getrennt und die organische Phase filtriert. Die organischen Phase besteht aus freien Fettsäuren (hauptsächlich < C₂₀) und Mono-, Di- und Triglyceriden von LCFA (> C₂₀). Die freien Fettsäuren (< C₂₀) werden vom Reaktionsgemisch mittels Kurzweg-Destillation (KD) unter Vakuum und bei Temperaturen von 140 bis 180 °C abdestilliert. Es wird ein Gemisch aus Mono-, Di-und Triglyceriden von LCFA (> C₂₀) erhalten.

In einem 1.000-ml-Mehrhalskolben mit Dephlegmator (Partialkondensator) und Destillationsbrücke werden 25 g einer (R)/(S)-3-Hydroxybuttersäure (3-BHB) in 95 g Essigsäure vorgelegt. Zu der Reaktionsmischung werden bei 80 °C unter N₂-Atomosphäre 90 g Essigsäureanhydrid innerhalb von einer Stunde zu getropft. Das Reaktionsgemisch wird bei 80 °C für weitere 4 bis 5 h gerührt. Zu der Reaktionsmischung werden bei 80 °C 2 g des oben hergestellten Gemisches aus Mono-, Di- und Triglyceriden von LCFA (> C₂₀) hinzugegeben und für 4 bis 5 h gerührt. Die entstehende 3-Acetoxybuttersäure bzw. Essigsäure wird im Vakuum (< 50 mbar) bei 100 bis 120 °C abdestilliert.

Das Reaktionsprodukt ist ein LCF / Acetyl-BHB-Triglycerid (3-Acetoxy-BHB-Triglycerid).

Spaltungsversuche (Spaltversuche) dieses Reaktionsprodukts in einem Magen- oder Darmmedium (FaSSGF-Medium, welches den Magen simuliert, oder FaSSIF-Medium, welches den Darmtrakt simuliert), jeweils in Anwesenheit oder in Abwesenheit von Pankreatin, belegen die Spaltung zu 3-BHB in freier Form. Diese Spaltungsversuche belegen, dass erfindungsgemäße Struktureinheiten auf Basis von Glyceriden von 3-Hydroxy- und/oder 3-Alkoxybuttersäuren enthaltende Lipide (hier konkret das erhaltene Reaktionsprodukt) effiziente Präkursoren bzw. Metabolite der freien 3-Hydroxybuttersäure bzw. deren Salze darstellen, insbesondere im Hinblick auf ihre beabsichtigte Wirkung, welche zudem in physiologisch verträglicher bzw. physiologisch kompatibler Form vorliegen.

### Beispiel 6:

### Herstellung von gemischten Long Chain Fatty Acid (LCFA), Medium Chain Fatty Acid (MCFA) und 3-Acetoxvbuttersäure(Ac-BHB)-Triglyceriden aus Partialglyceriden und 3-Acetoxybuttersäure-Anhydriden

In einem 1.000-ml-Mehrhalskolben mit Dephlegmator (Partialkondensator) und Destillationsbrücke werden 500 g eines langkettigen, raffinierten, gebleichten und desodorisierten Fischöls (EPA / DHA Gehalt 20 bis 50 %, Verhältnis 50 : 50) mit 250 g Wasser 0,4 g Enzym Lipase bei 40 °C unter Rühren und N₂-Atmosphäre 8 bis 12 h selektiv gespalten. Es werden hauptsächlich Fettsäuren < C₂₀ abgespalten. Danach werden die Phasen getrennt und die organische Phase filtriert. Die organischen Phase besteht aus freien Fettsäuren (hauptsächlich < C₂₀) und Mono-, Di- und Triglyceriden von LCFA (> C₂₀). Die freien Fettsäuren (< C₂₀) werden vom Reaktionsgemisch mittels Kurzweg-Destillation (KD) unter Vakuum und bei Temperaturen von 140 bis 180 °C abdestilliert. Es wird ein Gemisch aus Mono-, Di- und Triglyceriden von LCFA (> C₂₀) erhalten.

In einem 1.000-ml-Mehrhalskolben mit Dephlegmator (Partialkondensator) und Destillationsbrücke werden 25 g einer (R)/(S)-3-Hydroxybuttersäure (3-BHB) in 95 g Essigsäure vorgelegt. Zu der Reaktionsmischung werden bei 80 °C unter N₂-Atomosphäre 90 g Essigsäureanhydrid innerhalb von einer Stunde zu getropft. Das Reaktionsgemisch wird bei 80 °C für weitere 4 bis 5 h gerührt. Zu der Reaktionsmischung werden bei 80 °C 30 g des oben hergestellten Gemisches aus Mono-, Di- und Triglyceriden von LCFA (> C₂₀) und 7,5 g eines mittelkettigen Mono-/Di-Glycerids (Cs / C₁₀-Fettsäuren, Verhältnis 60 % / 40 %; Glyceridverteilung: Mono-Ester 30 bis 70 %, Di-Ester 10 bis 30 %, Tri-Ester 1 bis 5 %) hinzugegeben und für 4 bis 5 h gerührt. Die entstehende 3-Acetoxybuttersäure wird im Vakuum (< 50 mbar) bei 100 bis 120 °C abdestilliert. Das verbleibende Reaktionsprodukt wird nun mit immobilisiertem Enzym (CALB) bei 40 °C unter Rühren und N₂-Atmosphäre 8 bis 12 h randomisiert.

Das Reaktionsprodukt ist ein LCF / MCF / Acetyl-BHB-Triglycerid (3-Acetoxy-BHB-Triglycerid).

Spaltungsversuche (Spaltversuche) dieses Reaktionsprodukts in einem Magen- oder Darmmedium (FaSSGF-Medium, welches den Magen simuliert, oder FaSSIF-Medium, welches den Darmtrakt simuliert), jeweils in Anwesenheit oder in Abwesenheit von Pankreatin, belegen die Spaltung zu 3-BHB in freier Form. Diese Spaltungsversuche belegen, dass erfindungsgemäße Struktureinheiten auf Basis von Glyceriden von 3-Hydroxy- und/oder 3-Alkoxybuttersäuren enthaltende Lipide (hier konkret das erhaltene Reaktionsprodukt) effiziente Präkursoren bzw. Metabolite der freien 3-Hydroxybuttersäure bzw. deren Salze darstellen, insbesondere im Hinblick auf ihre beabsichtigte Wirkung, welche zudem in physiologisch verträglicher bzw. physiologisch kompatibler Form vorliegen.

### Beispiel 7:

### Herstellung von gemischten Long Chain Fatty Acid (LCFA), Medium Chain Fatty Acid (MCFA) und 3-Hydroxybuttersäure(3-BHB)-Trialyceriden aus Partialglyceriden

In einem 1.000-ml-Mehrhalskolben mit Dephlegmator (Partialkondensator) und Destillationsbrücke werden 500 g eines langkettigen, raffinierten, gebleichten und desodorisierten Fischöls (EPA / DHA-Gehalt 20 bis 50 %, Verhältnis 50:50) mit 250 g Wasser 0,4 g Enzym Lipase bei 40 °C unter Rühren und N₂-Atmosphäre 8 bis 12 h selektiv gespalten. Es werden hauptsächlich Fettsäuren < C₂₀ abgespalten. Danach werden die Phasen getrennt und die organische Phase filtriert. Die organischen Phase besteht aus freien Fettsäuren (hauptsächlich < C20) und Mono-, Di- und Triglyceriden von LCFA (> C₂₀). Die freien Fettsäuren (< C₂₀) werden vom Reaktionsgemisch mittels Kurzweg-Destillation (KD) unter Vakuum und bei Temperaturen von 140 bis 180 °C ab destilliert. Es wird ein Gemisch aus Mono-, Di- und Triglyceriden von LCFA (> C₂₀) erhalten.

In einem 250-ml-Mehrhalskolben mit Dephlegmator (Partialkondensator) und Destillationsbrücke werden 50 g des oben hergestellten Gemisches aus Mono-, Di- und Triglyceriden von LCFA (> C₂₀), 25 g eines mittelkettigen Mono-, Di-Glycerids (C₈ / C₁₀-Fettsäuren Verhältnis 60 % / 40 %; Glyceridverteilung: Mono-Ester 30 bis 70 %, Di-Ester 10 bis 30 %, Triglycerid 1 bis 5 %) und 35 g (R)/(S)-3-Hydroxybuttersäureethylester (3-BHB-Ethylester) bei 50 bis 70 °C, unter Vakuum (< 500 mbar) mittels 0,85 g immobilisiertem Enzym (CALB) umgeestert.

Das Reaktionsprodukt ist ein LCF / MCF / 3-BHB-Triglycerid.

Spaltungsversuche (Spaltversuche) dieses Reaktionsprodukts in einem Magen- oder Darmmedium (FaSSGF-Medium, welches den Magen simuliert, oder FaSSIF-Medium, welches den Darmtrakt simuliert), jeweils in Anwesenheit oder in Abwesenheit von Pankreatin, belegen die Spaltung zu 3-BHB in freier Form. Diese Spaltungsversuche belegen, dass erfindungsgemäße Struktureinheiten auf Basis von Glyceriden von 3-Hydroxy- und/oder 3-Alkoxybuttersäuren enthaltende Lipide (hier konkret das erhaltene Reaktionsprodukt) effiziente Präkursoren bzw. Metabolite der freien 3-Hydroxybuttersäure bzw. deren Salze darstellen, insbesondere im Hinblick auf ihre beabsichtigte Wirkung, welche zudem in physiologisch verträglicher bzw. physiologisch kompatibler Form vorliegen.

### Beispiel 8:

### Herstellung von 3-Acetoxybuttersäureethylester (3-Ac-BHB-Ethylester) und nachfolgende Umsetzung

In einem 250-ml-Mehrhalskolben mit Dephlegmator (Partialkondensator) und Destillationsbrücke werden 50 g (R)/(S)-3-Hydroxybuttersäureethylester (racemischer 3-BHB-Ethylester) und 55 g Essigsäureanhydrid vorgelegt. Das Reaktionsgemisch wird bei 100 °C unter Rühren und unter Rückfluss für 10 h zur Reaktion gebracht. Danach werden die entstandene Essigsäure sowie überschüssiges Essigsäureanhydrid unter Vakuum abdestilliert. Es wird ein 3-Acetoxybuttersäureethylester mit 98 % Reinheit erhalten. Die Charakterisierung erfolgt mittels Gaschromatographie (GC) und GC-MS-Analyse (Gaschromatographie mit Massenspektrometrie-Kopplung). Die Ergebnisse des Umsatz/Zeit-Verlaufs sind in der nachfolgenden Tabelle zusammengefasst.

| **Zeit / h** | **Essigsäureanhydrid / %** | **3-BHB-Ethylester / %** | **3-Ac-BHB-Ethylester (Produkt) / %** | **unbekannt** / **%** | **Kommentar** |
|---|---|---|---|---|---|
| 1 | 28,8 | 38,9 | 31,3 | 1 | 80 °C |
| 3 | 23,1 | 26,1 | 49,5 | 1,3 | - |
| 6 | 18,6 | 15,9 | 63,9 | 1,6 | Temperaturerhöhung auf 100 °C |
| 9 | 12,4 | 3,2 | 82,6 | 1,8 | - |
| 12 | 11,5 | 1,3 | 85,6 | 1,6 | - |
| - | 0 | 0,6 | 97,7 | 1,7 | nach Destillation |

Die weitere Umsetzung von 3-Acetoxybuttersäureethylester (3-Ac-BHB-Ethylester) und Anwendungsversuche sind nachfolgend geschildert: Im Weiteren kann gezeigt werden, dass der auf diese Weise erhaltene 3-Acetoxybuttersäureethylester mit Enzym als Katalysator (z. B. immobilisiertes Enzym, wie z. B. CALB-Lipase auf Polymerträger, abgeleitet von *Candida antarctica,* z. B. Novozym^{®}435 von der Fa. Sigma-Aldrich bzw. Merck oder Lipozym^{®}435 von der Fa. Strem Chemicals, Inc.) in einer Umesterung zu Glyceriden als Edukt eigesetzt werden kann (Umsetzung bei 50 bis 70 °C, 24 h 1 Gew.-% Enzym). Als weiteres Edukt wird Triacetin eingesetzt; da dieses bereits Acetylgruppen enthält, bilden sich bei einer etwaigen Umesterung an der bereits acetylierten OH-Gruppe des 3-BHB-Ethylesters keine unerwünschten Nebenprodukte. Als Koppelprodukt bildet sich nur Essigsäureethylester (Ethylacetat), welcher ohne Weiteres entfernt werden kann. Es entsteht ein Gemisch von Mono-, Di- und Triglyceriden der 3-Acetoxybuttersäure. Dieses Produkt ist ein Analogon bzw. eine Modellsubstanz zu den erfindungsgemäßen Produkten.

Spaltungsversuche (Spaltversuche) dieses Gemischs in einem Magen- oder Darmmedium (FaSSGF-Medium, welches den Magen simuliert, oder FaSSIF-Medium, welches den Darmtrakt simuliert), jeweils in Anwesenheit oder in Abwesenheit von Pankreatin, belegen die Spaltung zu 3-BHB in freier Form (Spaltungskaskade von Triglycerid über Diglycerid zu Monoglycerid zu freier 3-BHB). Diese Spaltungsversuche belegen, dass auch die Glyceride der verkappten (blockierten) 3-Hydroxybuttersäure bzw. deren Salze effiziente Präkursoren bzw. Metabolite der freien Hydroxybuttersäure bzw. deren Salze darstellen, insbesondere im Hinblick auf ihre beabsichtigte Wirkung, welche zudem in physiologisch verträglicher bzw. physiologisch kompatibler Form vorliegen.

### Beispiel 9:

### Weitere Herstellungsbeispiele: Carbonsäureanhydrid-Synthese (Edukt)

Nach dem beschriebenen Carbonsäureanhydrid-Herstellungsverfahren werden zunächst jeweils die Carbonsäureanhydride von Heptansäure (C₇-Säure), Laurinsäure (C₁₂-Säure) und Ölsäure (C₁₈-Säure) hergestellt.

Für die Herstellung des Carbonsäureanhydrids von Heptansäure (C₇-Säure) werden in einem 2.000-ml-Mehrhalskolben mit Dephlegmator (Partialkondensator) und Destillationsbrücke 860 g Heptansäure vorgelegt und bei 90 °C 445 g Essigsäureanhydrid unter Rühren zugetropft. Das Reaktionsgemisch wird danach bei 130 °C unter Rückfluss für 6 h unter Rühren zur Reaktion gebracht. Danach werden die entstandene Essigsäure sowie das überschüssige Essigsäureanhydrid unter Vakuum abdestilliert. Es wird das Heptansäureanhydrid erhalten. Die Charakterisierung erfolgt mittels GC und GC-MS.

In entsprechender Weise werden die Carbonsäureanhydride von Laurinsäure (C₁₂-Säure) und Ölsäure (C₁₈-Säure) hergestellt.

Nachfolgend wird - entsprechend der zuvor beschriebenen Herstellung von 3-Acetoxybuttersäureethylester - das betreffende Carbonsäureanhydrid mit 3-BHB-Ethylester umgesetzt, so dass jeweils die in 3-Position mit dem Carbonsäureanhydrid verkappten 3-BHB-Ethylester resultieren. Diese können als Edukte in den beschriebenen Synthesen eingesetzt werden.

### Weitere physiologische Anwendungsversuche:in-vitro-Verdauversuche Verdauversuche (Spalt- bzw. Spaltunasversuche) von erfindunasaemäßen Verbindungen

Mittels Spaltungsversuchen wird gezeigt, dass erfindungsgemäß hergestellte Reaktionsprodukte bzw. deren Gemische (Reaktionsprodukte aus den vorherigen Beispielen 1 bis 7) im menschlichen gastrointestinalen Trakt gespalten werden können. Die wie zuvor beschrieben erhaltenen und aufgereinigten Reaktionsprodukte aus den Beispielen 1 bis 7 werden den nachfolgend beschriebenen Spaltungsversuchen unterzogen.

Für die Spaltungsversuche unter körpernahen Bedingungen werden zwei Medien untersucht:
- FaSSGF, welches den Magen simuliert
- FaSSIF, welches den Darmtrakt simuliert

Beide Medien stammen von der Firma Biorelevant^{®}, Ltd., Großbritannien. Zusätzlich wird in einigen Experimenten beiden Medien jeweils Schweine-Pankrease zugesetzt (Panzytrat^{®} 40.000, Fa. Allergan).

Die Ergebnisse der Spaltungsversuche in einem FaSSGF- bzw. FaSSIF-Medium mit Panzytrat^{®} und ohne Panzytrat^{®} (jeweils 35 °C, 24 h) zeigen, dass alle Proben unter FaSSGF-Bedingungen mit Panzytrat^{®} und ohne Panzytrat^{®} hydrolysieren; dies liegt hauptsächlich am niedrigen pH-Wert (pH = 1,6). Bei FaSSIF-Bedingungen findet eine geringere Umsetzung unter Verwendung von Panzytrat^{®} statt.

Bei allen Experimenten ist zu erkennen, dass die gewünschte freie Säure 3-BHB generiert wird. Der Umsatz/Zeit-Verlauf der wässrigen Spaltung der erfindungsgemäßen Verbindungen, einschließlich Zunahme der Säurezahl über die Zeit, belegt die gewünschte Zersetzung der Edukte zu der freien Säure (3-BHB). Dies wird durch entsprechende Analytik bestätigt.

Die zuvor geschilderten Spaltungsversuche belegen, dass die erfindungsgemäßen, Struktureinheiten auf Basis von Glyceriden von 3-Hydroxy- und/oder 3-Alkoxybuttersäuren enthaltenden Lipide effiziente Präkursoren bzw. Metabolite der freien Hydroxybuttersäure bzw. deren Salze darstellen, insbesondere im Hinblick auf ihre beabsichtigte Wirkung, und zudem in physiologisch verträglicher bzw. physiologisch kompatibler Form vorliegen.

## Patentansprüche

1. Verfahren zur Herstellung von Lipiden, welche Struktureinheiten auf Basis von Glyceriden von 3-Hydroxy- und/oder 3-Alkoxybuttersäuren enthalten,
wobei mindestens ein Glycerid der allgemeinen Formel (I)
R¹O-CH₂-CH(OR²-CH₂-OR³ (I)
wobei in der allgemeinen Formel (I) die Reste R¹, R² und R³, gleich oder verschieden, jeweils unabhängig voneinander
• Wasserstoff,
• einen Rest C₁-C₁₁-Alkyl-C(O)- , insbesondere C₂-C₁₁-Alkyl-C(O)- ,
• einen Rest C₁₂-C₂₉-Alkyl-C(O)- , insbesondere C₁₉-C₂₉-Alkyl-C(O)- , darstellen, jedoch mit der Maßgabe, dass mindestens einer, vorzugsweise mindestens zwei der Reste R¹, R² und R³ keinen Wasserstoff darstellen,
mit mindestens einem 3-Hydroxy- und/oder 3-Alkoxybuttersäure-Derivat der allgemeinen Formel (II)
CH₃-CH(OR⁴)-CH₂-C(O)OR⁵ (II)
wobei in der allgemeinen Formel (II)
• der Rest R⁴ Wasserstoff oder einen Rest C₁-C₃₀-Alkyl-C(O)- , insbesondere C₁-C₂₁-Alkyl-C(O)- , vorzugsweise C₃-C₂₁-Alkyl-C(O)- , darstellt,
• der Rest R⁵ Wasserstoff oder ein C₁-C₄-Alkyl, insbesondere ein C₁-C₄-Alkyl, bevorzugt Methyl oder Ethyl, besonders bevorzugt Ethyl, darstellt,
umgesetzt wird,
wobei die Umsetzung in Abwesenheit von Lösemitteln und/oder ohne jedwedes Lösemittel durchgeführt wird und
wobei die Umsetzung in Gegenwart eines Enzyms als Katalysator durchgeführt wird,
so dass als Reaktionsprodukt ein Gemisch von mindestens zwei voneinander verschiedenen, Struktureinheiten auf Basis von Glyceriden von 3-Hydroxy- und/oder 3-Alkoxybuttersäuren enthaltenden Lipiden der allgemeinen Formel (III)
R⁶O-CH₂-CH(OR⁷)-CH₂-OR⁸ (III)
wobei in der allgemeinen Formel (III) die Reste R⁶, R⁷ und R⁸, gleich oder verschieden, jeweils unabhängig voneinander
• einen Rest C₁-C₁₁-Alkyl-C(O)-, insbesondere C₂-C₁₁-Alkyl-C(O)-,
• einen Rest C₁₂-C₂₉-Alkyl-C(O)-, insbesondere C₁₉-C₂₉-Alkyl-C(O)-,
• einen Rest CH₃-CH(OR⁴)-CH₂-C(O)-, worin der Rest R⁴ die zuvor angegebene Bedeutung hat,
darstellen, jedoch mit der Maßgabe, dass einer oder zwei der Reste R⁶, R⁷ und R⁸ einen Rest CH₃-CH(OR⁴)-CH₂-C(O)- darstellt,
erhalten wird.

2. Verfahren nach Anspruch 1,
wobei in der allgemeinen Formel (II)
• der Rest R⁴ Wasserstoff oder einen Rest vom Typ lineares (geradkettiges) oder verzweigtes, gesättigtes oder ein- oder mehrfach ungesättigtes C₁-C₃₀-Alkyl-C(O)-, insbesondere lineares (geradkettiges) oder verzweigtes, gesättigtes oder ein- oder mehrfach ungesättigtes C₁-C₂₁-Alkyl-C(O)-, vorzugsweise lineares (geradkettiges) oder verzweigtes, gesättigtes oder ein- oder mehrfach ungesättigtes C₃-C₂₁-Alkyl-C(O)-, darstellt,
• der Rest R⁵ ein C₁-C₄-Alkyl, insbesondere ein C₁-C₄-Alkyl, bevorzugt Methyl oder Ethyl, besonders bevorzugt Ethyl, darstellt; und/oder
wobei in der allgemeinen Formel (II)
• der Rest R⁴ Wasserstoff oder einen Rest vom Typ lineares (geradkettiges) oder verzweigtes, gesättigtes oder ein- oder mehrfach ungesättigtes C₁-C₃₀-Alkyl-C(O)-, insbesondere lineares (geradkettiges) oder verzweigtes, gesättigtes oder ein- oder mehrfach ungesättigtes C₁-C₂₁-Alkyl-C(O)-, vorzugsweise lineares (geradkettiges) oder verzweigtes, gesättigtes oder ein- oder mehrfach ungesättigtes C₃-C₂₁-Alkyl-C(O)-, darstellt,
• der Rest R⁵ Ethyl darstellt; und/oder
wobei in der allgemeinen Formel (II) der Rest R⁴ Wasserstoff und der Rest R⁵ Ethyl darstellt; und/oder
wobei als 3-Hydroxy- und/oder 3-Alkoxybuttersäure-Derivat der allgemeinen Formel (II) 3-Hydroxybuttersäureethylester (Ethyl-3-hydroxybutyrat) der Formel CH₃-CH(OH)-CH₂-C(O)OC₂H₅ eingesetzt wird; und/oder
wobei das 3-Hydroxy- und/oder 3-Alkoxybuttersäure-Derivat der allgemeinen Formel (II) in racemischer Form oder in Form des (R)-Enantiomers eingesetzt wird; insbesondere wobei die (R)-Konfiguration auf das Kohlenstoffatom in 3-Position bezogen ist und/oder insbesondere wobei die (R)-Konfiguration auf das den OR⁴-Rest tragende Kohlenstoffatom bezogen ist.

3. Verfahren nach einem der vorangehenden Ansprüche,
wobei der Katalysators nach Umsetzung rezykliert wird; und/oder
wobei das Enzym ausgewählt wird aus Synthetasen (Ligasen), Katalasen, Esterasen, Lipasen und deren Kombinationen; und/oder
wobei das Enzym sich ableitet von *Candida antarctica, Mucor miehei* (*Rhizomucor miehei), Thermomyces lanuginosus, Candida rugosa, Aspergillus oryzae, Pseudomonas cepacia, Pseudomonas fluorescens, Rhizopus delemar* und *Pseudomonas* sp. sowie deren Kombinationen, vorzugsweise von *Candida antarctica, Mucor miehei* (*Rhizomucor miehei*) und *Thermomyces lanuginosus;* und/oder
wobei das Enzym in immobilisierter Form, insbesondere immobilisiert auf einem Träger, vorzugsweise auf einem polymeren Träger, bevorzugt auf einem polymeren organischen Träger, besonders bevorzugt mit hydrophoben Eigenschaften, ganz besonders bevorzugt auf einem poly(meth)acrylharzbasierten Träger, eingesetzt wird; und/oder
wobei das Enzym nach der Umsetzung rezykliert wird; und/oder
wobei die Umsetzung in Gegenwart eines Enzyms als Katalysator bei Temperaturen im Bereich von 10 °C bis 80 °C, insbesondere im Bereich von 20 °C bis 80 °C, vorzugsweise im Bereich von 25 °C bis 75 °C, besonders bevorzugt im Bereich von 45 °C bis 75 °C, ganz besonders bevorzugt im Bereich von 50 °C bis 70 °C, durchgeführt wird; und/oder
wobei das Enzym in Mengen, bezogen auf die Gesamtmenge der Ausgangsverbindungen (I) und (II), im Bereich von 0,001 Gew.-% bis 20 Gew.-%, insbesondere im Bereich von 0,01 Gew.-% bis 15 Gew.-%, vorzugsweise im Bereich von 0,1 Gew.-% bis 15 Gew.-%, bevorzugt im Bereich von 0,5 Gew.-% bis 10 Gew.-%, eingesetzt wird; und/oder
wobei die Umsetzung in Gegenwart eines Enzyms als Katalysator bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt wird.

4. Verfahren nach einem der vorangehenden Ansprüche,
wobei für den Fall, dass in der allgemeinen Formel (II) der Rest R⁵ Wasserstoff bezeichnet, anstelle der freien Säure deren Anhydrid der allgemeinen Formel (IIa)
[CH₃-CH(OR⁴)-CH₂-C(O)]₂O (IIa)
wobei in der allgemeinen Formel (IIa) der Rest R⁴ die zuvor angegebene Bedeutung hat,
eingesetzt wird.

5. Gemisch, umfassend mindestens zwei voneinander verschiedene, Struktureinheiten auf Basis von Glyceriden von 3-Hydroxy- und/oder 3-Alkoxybuttersäuren enthaltende Lipide der allgemeinen Formel (III)
R⁶O-CH₂-CH(OR⁷)-CH₂-OR⁸ (III)
wobei in der allgemeinen Formel (III) die Reste R⁶, R⁷ und R⁸, gleich oder verschieden, jeweils unabhängig voneinander
• einen Rest C₁-C₁₁-Alkyl-C(O)- , insbesondere C₂-C₁₁-Alkyl-C(O)- ,
• einen Rest C₁₂-C₂₉-Alkyl-C(O)- , insbesondere C₁₉-C₂₉-Alkyl-C(O)- ,
• einen Rest CH₃-CH(OR⁴)-CH₂-C(O)- , worin der Rest R⁴ die zuvor angegebene Bedeutung hat, insbesondere wobei der Rest R⁴ Wasserstoff oder einen Rest C₁-C₃₀-Alkyl-C(O)- , insbesondere C₁-C₂₁-Alkyl-C(O)- , vorzugsweise C₃-C₂₁-Alkyl-C(O)- , darstellt,
darstellen, jedoch mit der Maßgabe, dass ein oder zwei der Reste R⁶, R⁷ und R⁸ einen Rest CH₃-CH(OR⁴)-CH₂-C(O)- darstellt.

6. Gemisch nach Anspruch 5,
wobei in der allgemeinen Formel (III) die Reste R⁶, R⁷ und R⁸, gleich oder verschieden, jeweils unabhängig voneinander
• einen Rest vom Typ lineares (geradkettiges) oder verzweigtes, gesättigtes oder ein- oder mehrfach ungesättigtes C₁-C₁₁-Alkyl-C(O)- , insbesondere lineares (geradkettiges) oder verzweigtes, gesättigtes oder ein- oder mehrfach ungesättigtes C₂-C₁₁-Alkyl-C(O)- ,
• einen Rest vom Typ lineares (geradkettiges) oder verzweigtes, gesättigtes oder ein- oder mehrfach ungesättigtes C₁₂-C₂₉-Alkyl-C(O)- , insbesondere lineares (geradkettiges) oder verzweigtes, gesättigtes oder ein- oder mehrfach ungesättigtes C₁₉-C₂₉-Alkyl-C(O) - ,
• einen Rest vom Typ lineares (geradkettiges) oder verzweigtes, gesättigtes oder ein- oder mehrfach ungesättigtes CH₃-CH(OR⁴)-CH₂-C(O)- , worin der Rest R⁴ die zuvor angegebene Bedeutung hat, insbesondere wobei der Rest R⁴ Wasserstoff oder einen Rest C₁-C₃₀-Alkyl-C(O)- , insbesondere C₁-C₂₁-Alkyl-C(O)- , vorzugsweise C₃-C₂₁-Alkyl-C(O)- , darstellt,
darstellen, jedoch mit der Maßgabe, dass mindestens einer der Reste R⁶, R⁷ und R⁸ einen Rest CH₃-CH(OR⁴)-CH₂-C(O)- darstellt.

7. Gemisch nach Anspruch 5 oder 6,
wobei in der allgemeinen Formel (III) die Reste R⁶, R⁷ und R⁸, gleich oder verschieden, jeweils unabhängig voneinander
• einen Rest C₁-C₁₁-Alkyl-C(O)- , insbesondere C₂-C₁₁-Alkyl-C(O)- ,
• einen Rest C₁₂-C₂₉-Alkyl-C(O)- , insbesondere C₁₉-C₂₉-Alkyl-C(O)- ,
• einen Rest CH₃-CH(OR⁴)-CH₂-C(O)- , worin der Rest R⁴ die zuvor angegebene Bedeutung hat, insbesondere wobei der Rest R⁴ Wasserstoff oder einen Rest C₁-C₃₀-Alkyl-C(O)- , insbesondere C₁-C₂₁-Alkyl-C(O)- , vorzugsweise C₃-C₂₁-Alkyl-C(O)- , darstellt,
darstellen, jedoch mit der Maßgabe, dass zwei Reste R⁶, R⁷ und R⁸ einen Rest CH₃-CH(OR⁴)-CH₂-C(O)- darstellen.

8. Gemisch nach einem der Ansprüche 5 bis 7,
wobei in der allgemeinen Formel (III) die Reste R⁶, R⁷ und R⁸, gleich oder verschieden, jeweils unabhängig voneinander
• einen Rest C₁-C₁₁-Alkyl-C(O)- , insbesondere C₂-C₁₁-Alkyl-C(O)- ,
• einen Rest CH₃-CH(OR⁴)-CH₂-C(O)- , worin der Rest R⁴ die zuvor angegebene Bedeutung hat, insbesondere wobei der Rest R⁴ Wasserstoff oder einen Rest C₁-C₃₀-Alkyl-C(O)- , insbesondere C₁-C₂₁-Alkyl-C(O)- , vorzugsweise C₃-C₂₁-Alkyl-C(O) - , darstellt,
darstellen, jedoch mit der Maßgabe, dass zwei Reste R⁶, R⁷ und R⁸ einen Rest CH₃-CH(OR⁴) - CH₂ - C(O) - darstellen.

9. Gemisch nach einem der Ansprüche 5 bis 8,
wobei in der allgemeinen Formel (III) die Reste R⁶, R⁷ und R⁸, gleich oder verschieden, jeweils unabhängig voneinander
• einen Rest C₁₂-C₂₉-Alkyl- C(O) - , insbesondere C₁₉-C₂₉-Alkyl- C(O) - ,
• einen Rest CH₃-CH(OR⁴)-CH₂-C(O)- , worin der Rest R⁴ die zuvor angegebene Bedeutung hat, insbesondere wobei der Rest R⁴ Wasserstoff oder einen Rest C₁-C₃₀-Alkyl-C(O)- , insbesondere C₁-C₂₁-Alkyl-C(O)- , vorzugsweise C₃-C₂₁-Alkyl-C(O)- , darstellt,
darstellen, jedoch mit der Maßgabe, dass zwei Reste R⁶, R⁷ und R⁸ einen Rest CH₃-CH(OR⁴)-CH₂-C(O)- darstellen.

10. Gemisch nach einem der Ansprüche 5 bis 9,
wobei in der allgemeinen Formel (III) die Reste R⁶, R⁷ und R⁸, gleich oder verschieden, jeweils unabhängig voneinander
• einen Rest C₁-C₁₁-Alkyl-C(O)- , insbesondere C₂-C₁₁-Alkyl-C(O)- ,
• einen Rest C₁₂-C₂₉-Alkyl-C(O)- , insbesondere C₁₉-C₂₉-Alkyl-C(O)- ,
• einen Rest CH₃-CH(OR⁴)-CH₂-C(O)- , worin der Rest R⁴ die zuvor angegebene Bedeutung hat, insbesondere wobei der Rest R⁴ Wasserstoff oder einen Rest C₁-C₃₀-Alkyl-C(O)- , insbesondere C₁-C₂₁-Alkyl-C(O)- , vorzugsweise C₃-C₂₁-Alkyl-C(O)- , darstellt,
darstellen, jedoch mit der Maßgabe, dass einer der Reste R⁶, R⁷ und R⁸ einen Rest CH₃-CH(OR⁴)-CH₂-C(O)- darstellt.

11. Gemisch nach einem der Ansprüche 5 bis 10,
wobei in der allgemeinen Formel (III) die Reste R⁶, R⁷ und R⁸, gleich oder verschieden, jeweils unabhängig voneinander
• einen Rest C₁-C₁₁-Alkyl-C(O)- , insbesondere C₂-C₁₁-Alkyl-C(O)- ,
• einen Rest CH₃-CH(OR⁴)-CH₂-C(O)- , worin der Rest R⁴ die zuvor angegebene Bedeutung hat, insbesondere wobei der Rest R⁴ Wasserstoff oder einen Rest C₁-C₃₀-Alkyl- C(O) - , insbesondere C₁-C₂₁-Alkyl-C(O)- , vorzugsweise C₃-C₂₁-Alkyl-C(O)- , darstellt,
darstellen, jedoch mit der Maßgabe, dass einer der Reste R⁶, R⁷ und R⁸ einen Rest CH₃-CH(OR⁴)-CH₂-C(O)- darstellt.

12. Gemisch nach einem der Ansprüche 5 bis 11,
wobei in der allgemeinen Formel (III) die Reste R⁶, R⁷ und R⁸, gleich oder verschieden, jeweils unabhängig voneinander
• einen Rest C₁₂-C₂₉-Alkyl-C(O)- , insbesondere C₁₉-C₂₉-Alkyl-C(O)- ,
• einen Rest CH₃-CH(OR⁴)-CH₂-C(O)- , worin der Rest R⁴ die zuvor angegebene Bedeutung hat, insbesondere wobei der Rest R⁴ Wasserstoff oder einen Rest C₁-C₃₀-Alkyl-C(O)- , insbesondere C₁-C₂₁-Alkyl-C(O)- , vorzugsweise C₃-C₂₁-Alkyl-C(O)- , darstellt,
darstellen, jedoch mit der Maßgabe, dass einer der Reste R⁶, R⁷ und R⁸ einen Rest CH₃-CH(OR⁴)-CH₂-C(O)- darstellt.

13. Gemisch nach einem der Ansprüche 5 bis 12,
wobei in der allgemeinen Formel (III) die Reste R⁶, R⁷ und R⁸, gleich oder verschieden, jeweils unabhängig voneinander
• einen Rest C₁-C₁₁-Alkyl-C(O)- , insbesondere C₂-C₁₁-Alkyl-C(O)- ,
• einen Rest C₁₂-C₂₉-Alkyl- C(O) - , insbesondere C₁₉-C₂₉-Alkyl-C(O)- ,
• einen Rest CH₃- CH(OR⁴) - CH₂ - C(O) - , worin der Rest R⁴ die zuvor angegebene Bedeutung hat, insbesondere wobei der Rest R⁴ Wasserstoff oder einen Rest C₁-C₃₀-Alkyl- C(O) - , insbesondere C₁-C₂₁-Alkyl-C(O)- , vorzugsweise C₃-C₂₁-Alkyl-C(O)- , darstellt,
darstellen, jedoch mit der Maßgabe, dass einer der Reste R⁶, R⁷ und R⁸ einen Rest CH₃-CH(OR⁴)-CH₂-C(O)- darstellt und dass einer der Reste R⁶, R⁷ und R⁸ einen Rest C₁-C₁₁-Alkyl-C(O)- , insbesondere C₂-C₁₁-Alkyl-C(O)- , darstellt und dass einer der Reste R⁶, R⁷ und R⁸ einen Rest C₁₂-C₂₉-Alkyl-C(O)- , insbesondere C₁₉-C₂₉-Alkyl-C(O)- , darstellt.

14. Gemisch, umfassend mindestens drei voneinander verschiedene, Struktureinheiten auf Basis von Glyceriden von 3-Hydroxy- und/oder 3-Alkoxybuttersäuren enthaltende Lipide der allgemeinen Formel (III), wie in den vorhergehenden Ansprüchen definiert.

15. Pharmazeutische Zusammensetzung, insbesondere Arzneimittel oder Medikament, umfassend ein Gemisch gemäß einem der Ansprüche 5 bis 14.

16. Pharmazeutische Zusammensetzung nach Anspruch 15 zur prophylaktischen und/oder therapeutischen Behandlung bzw. zur Verwendung bei der prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

17. Gemisch gemäß einem der Ansprüche 5 bis 14 zur Verwendung bei der prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

18. Verwendung eines Gemischs gemäß einem der Ansprüche 5 bis 14 zur Herstellung eines Arzneimittels zur prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

19. Nahrungsmittel- und/oder Lebensmittelerzeugnis, umfassend ein Gemisch gemäß einem der Ansprüche 5 bis 14.

20. Verwendung eines Gemischs gemäß einem der Ansprüche 5 bis 14 in einem Nahrungsmittel- und/oder Lebensmittelerzeugnis.

## Claims

1. A method for producing lipids comprising structural units based on glycerides of 3-hydroxybutyric and/or 3-alkoxybutyric acids,
wherein at least one glyceride of the general formula (I)
R¹O-CH₂-CH(OR²)-CH₂-OR³ (I)
wherein in the general formula (I) the radicals R¹, R² and R³, identical or different, each independently of one another represent
• hydrogen,
• a radical C₁-C₁₁-alkyl-C(O)-, especially C₂-C₁₁-alkyl-C(O)- ,
• a radical C₁₂-C₂₉-alkyl-C(O)- , especially C₁₉-C₂₉-alkyl-C(O)- , however, with the proviso that at least one, preferentially at least two, of the radicals R¹, R² and R³ do not represent hydrogen,
is reacted
with at least one 3-hydroxybutyric and/or 3-alkoxybutyric acid derivative of the general formula (II)
CH₃-CH(OR⁴)-CH₂-C(O)OR⁵ (II)
wherein in the general formula (II)
• the radical R⁴ represents hydrogen or a radical C₁-C₃₀-alkyl-C(O)- , especially C₁-C₂₁-alkyl-C(O)- , preferentially C₃-C₂₁-alkyl-C(O)- ,
• the radical R⁵ represents hydrogen or C₁-C₄-alkyl, especially C₁-C₄-alkyl, preferably methyl or ethyl, more preferably ethyl,
wherein the reaction is carried out in the absence of solvents and/or without any solvent and
wherein the reaction is carried out in the presence of an enzyme as a catalyst,
so that, as a reaction product, there is obtained a mixture of at least two different lipids comprising structural units based on glycerides of 3-hydroxybutyric and/or 3-alkoxybutyric acids of the general formula (III)
R⁶O-CH₂-CH(OR⁷)-CH₂-OR⁸ (III)
wherein in the general formula (III) the radicals R⁶, R⁷ and R⁸, identical or different, each independently of one another represent
• a radical C₁-C₁₁-alkyl-C(O)- , especially C₂-C₁₁-alkyl-C(O)- ,
• a radical C₁₂-C₂₉-alkyl-C(O)- , especially C₁₉-C₂₉-alkyl-C(O)- ,
• a radical CH₃-CH(OR⁴)-CH₂-C(O)- , wherein the radical R⁴ has the meaning defined hereinabove,
however, with the proviso that one or two of the radicals R⁶, R⁷ and R⁸ represents a radical CH₃-CH(OR⁴)-CH₂-C(O)- .

2. The method according to claim 1,
wherein in the general formula (II)
• the radical R⁴ represents hydrogen or a radical of the type linear (straight-chain) or branched, saturated or mono- or polyunsaturated C₁-C₃₀-alkyl-C(O) - , especially linear (straight-chain) or branched, saturated or mono- or polyunsaturated C₁-C₂₁-alkyl-C(O)- , preferentially linear (straight-chain) or branched, saturated or mono- or polyunsaturated C₃-C₂₁-alkyl- C(O) - ,
• the radical R⁵ represents C₁-C₄-alkyl, especially C₁-C₄-alkyl, preferably methyl or ethyl, more preferably ethyl; and/or
wherein in the general formula (II)
• the radical R⁴ represents hydrogen or a radical of the type linear (straight-chain) or branched, saturated or mono- or polyunsaturated C₁-C₃₀-alkyl-C(O) - , especially linear (straight-chain) or branched, saturated or mono- or polyunsaturated C₁-C₂₁-alkyl-C(O)- , preferentially linear (straight-chain) or branched, saturated or mono- or polyunsaturated C₃-C₂₁-alkyl-C(O)- ,
• the radical R⁵ represents ethyl; and/or
wherein in the general formula (II) the radical R⁴ represents hydrogen and the radical R⁵ represents ethyl; and/or
wherein, as a 3-hydroxy- and/or 3-alkoxybutyric acid derivative of the general formula (II), 3-hydroxybutyric acid ethyl ester (ethyl 3-hydroxybutyrate) of the formula CH₃-CH(OH)-CH₂-C(O)OC₂H₅ is used; and/or
wherein the 3-hydroxybutyric and/or 3-alkoxybutyric acid derivative of the general formula (II) is used in racemic form or in the form of the (R)-enantiomer; especially wherein the (R)-configuration is based on the carbon atom in 3-position and/or especially wherein the (R)-configuration is based on the carbon atom carrying the OR⁴ radical.

3. The method according to any of the preceding claims,
wherein the catalyst is recycled after the reaction; and/or
wherein the reaction is carried out in the presence of an enzyme as a catalyst;
wherein the enzyme is selected from synthetases (ligases), catalases, esterases, lipases and combinations thereof; and/or
wherein the enzyme is derived from *Candida antarctica, Mucor miehei (Rhizomucor miehei), Thermomyces lanuginosus, Candida rugosa, Aspergillus oryzae, Pseudomonas cepacia, Pseudomonas fluorescens, Rhizopus delemar* and *Pseudomonas sp.* and combinations thereof, preferentially of *Candida antarctica, Mucor miehei (Rhizomucor miehei)* and *Thermomyces lanuginosus;* and/or
wherein the enzyme is used in immobilized form, especially immobilized on a carrier, preferentially on a polymeric carrier, preferably on a polymeric organic carrier, more preferably with hydrophobic properties, even more preferably on a poly(meth)acrylic resin-based carrier; and/or
wherein the enzyme is recycled after the reaction; and/or
wherein the reaction is carried out in the presence of an enzyme as a catalyst at temperatures in the range of from 10 °C to 80 °C, especially in the range of from 20 °C to 80 °C, preferentially in the range of from 25 °C to 75 °C, more preferably in the range of from 45 °C to 75 °C, even more preferably in the range of from 50 °C to 70 °C; and/or
wherein the enzyme is used in amounts, based on the total amount of starting compounds (I) and (II), in the range of from 0.001 % by weight to 20 % by weight, especially in the range of from 0.01 % by weight to 15 % by weight, preferentially in the range of from 0.1% by weight to 15% by weight, preferably in the range of from 0.5 % by weight to 10 % by weight; and/or
wherein the reaction is carried out in the presence of an enzyme as a catalyst at a pressure in the range of from 0.0001 bar to 10 bar, especially in the range of from 0.001 bar to 5 bar, preferentially in the range of from 0.01 bar to 2 bar, more preferably in the range of from 0.05 bar to 1 bar, even more preferably at about 1 bar.

4. The method according to any of the preceding claims,
wherein, in the case that in general formula (II) the radical R⁵ denotes hydrogen, its anhydride of the general formula (IIa)
[CH₃-CH(OR⁴)-CH₂-C(O)]₂O (IIa)
wherein in the general formula (IIa) the radical R⁴ has the meaning defined hereinabove,
is used instead of the free acid.

5. A mixture comprising at least two different lipids comprising structural units based on glycerides of 3-hydroxybutyric and/or 3-alkoxybutyric acids of the general formula (III)
R⁶O-CH₂-CH(OR⁷)-CH₂-OR⁸ (III)
wherein in the general formula (III) the radicals R⁶, R⁷ and R⁸, identical or different, each independently of one another represent
• a radical C₁-C₁₁-alkyl-C(O)- , especially C₂-C₁₁-alkyl-C(O)- ,
• a radical C₁₂-C₂₉-alkyl-C(O)- , especially C₁₉-C₂₉-alkyl-C(O)- ,
• a radical CH₃-CH(OR⁴)-CH₂-C(O)- , wherein the radical R⁴ has the meaning defined hereinabove, especially wherein the radical R⁴ represents hydrogen or a radical C₁-C₃₀-alkyl-C(O)- , especially C₁-C₂₁-alkyl-C(O)- , preferentially C₃-C₂₁-alkyl-C(O)- ,
however, with the proviso that one or two of the radicals R⁶, R⁷ and R⁸ represents a radical CH₃- CH(OR⁴) - CH₂ - C(O) - .

6. The mixture according to claim 5,
wherein in the general formula (III) the radicals R⁶, R⁷ and R⁸, identical or different, each independently of one another represent
• a radical of the type linear (straight-chain) or branched, saturated or mono- or polyunsaturated C₁-C₁₁-alkyl-C(O)- , especially linear (straight-chain) or branched, saturated or mono- or polyunsaturated C₂-C₁₁-alkyl-C(O)- ,
• a radical of the type linear (straight-chain) or branched, saturated or mono- or polyunsaturated C₁₂-C₂₉-alkyl- C(O) - , especially linear (straight-chain) or branched, saturated or mono- or polyunsaturated C₁₉-C₂₉-alkyl- C(O) - ,
• a radical of the type linear (straight-chain) or branched, saturated or mono- or polyunsaturated CH₃-CH(OR⁴)-CH₂-C(O)- , wherein the radical R⁴ has the meaning defined hereinabove, especially wherein the radical R⁴ represents hydrogen or a radical C₁-C₃₀-alkyl-C(O)-, especially C₁-C₂₁-alkyl- C(O) - , preferentially C₃-C₂₁-alkyl- C(O) - ,
however, with the proviso that at least one of the radicals R⁶, R⁷ and R⁸ represents a radical CH₃-CH(OR⁴)-CH₂-C(O)- .

7. The mixture according to claim 5 or 6,
wherein in the general formula (III) the radicals R⁶, R⁷ and R⁸, identical or different, each independently of one another represent
• a radical C₁-C₁₁-alkyl-C(O)- , especially C₂-C₁₁-alkyl-C(O)- ,
• a radical C₁₂-C₂₉-alkyl-C(O)- , especially C₁₉-C₂₉-alkyl- C(O) - ,
• a radical CH₃-CH(OR⁴)-CH₂-C(O)- , wherein the radical R⁴ has the meaning defined hereinabove, especially wherein the radical R⁴ represents hydrogen or a radical C₁-C₃₀-alkyl-C(O)- , especially C₁-C₂₁-alkyl-C(O)- , preferentially C₃-C₂₁-alkyl-C(O)- ,
however, with the proviso that two radicals R⁶, R⁷ and R⁸ represent a radical CH₃-CH(OR⁴)-CH₂-C(O)- .

8. The mixture according to any of claims 5 to 7,
wherein in the general formula (III) the radicals R⁶, R⁷ and R⁸, identical or different, each independently of one another represent
• a radical C₁-C₁₁-alkyl-C(O)- , especially C₂-C₁₁-alkyl-C(O)- ,
• a radical CH₃-CH(OR⁴)-CH₂-C(O)- , wherein the radical R⁴ has the meaning defined hereinabove, especially wherein the radical R⁴ represents hydrogen or a radical C₁-C₃₀-alkyl-C(O)- , especially C₁-C₂₁-alkyl-C(O)- , preferentially C₃-C₂₁-alkyl-C(O)- ,
however, with the proviso that two radicals R⁶, R⁷ and R⁸ represent a radical CH₃-CH(OR⁴)-CH₂-C(O)- .

9. The mixture according to any of claims 5 to 8,
wherein in the general formula (III) the radicals R⁶, R⁷ and R⁸, identical or different, each independently of one another represent
• a radical C₁₂-C₂₉-alkyl- C(O) - , especially C₁₉-C₂₉-alkyl- C(O) - ,
• a radical CH₃-CH(OR⁴)-CH₂-C(O)- , wherein the radical R⁴ has the meaning defined hereinabove, especially wherein the radical R⁴ represents hydrogen or a radical C₁-C₃₀-alkyl-C(O)- , especially C₁-C₂₁-alkyl-C(O)- , preferentially C₃-C₂₁-alkyl-C(O)- ,
however, with the proviso that two radicals R⁶, R⁷ and R⁸ represent a radical CH₃-CH(OR⁴)-CH₂-C(O)- .

10. The mixture according to any of claims 5 to 9,
wherein in the general formula (III) the radicals R⁶, R⁷ and R⁸, identical or different, each independently of one another represent
• a radical C₁-C₁₁-alkyl-C(O)- , especially C₂-C₁₁-alkyl-C(O)- ,
• a radical C₁₂-C₂₉-alkyl-C(O)- , especially C₁₉-C₂₉-alkyl-C(O)- ,
• a radical CH₃- CH(OR⁴) - CH₂ - C(O) - , wherein the radical R⁴ has the meaning defined hereinabove, especially wherein the radical R⁴ represents hydrogen or a radical C₁-C₃₀-alkyl-C(O)- , especially C₁-C₂₁-alkyl-C(O)- , preferentially C₃-C₂₁-alkyl-C(O)- ,
however, with the proviso that one of the radicals R⁶, R⁷ and R⁸ represents a radical CH₃-CH(OR⁴)-CH₂-C(O)- .

11. The mixture according to any of claims 5 to 10,
wherein in the general formula (III) the radicals R⁶, R⁷ and R⁸, identical or different, each independently of one another represent
• a radical C₁-C₁₁-alkyl-C(O)- , especially C₂-C₁₁-alkyl-C(O)- ,
• a radical CH₃-CH(OR⁴)-CH₂-C(O)- , wherein the radical R⁴ has the meaning defined hereinabove, especially wherein the radical R⁴ represents hydrogen or a radical C₁-C₃₀-alkyl-C(O)- , especially C₁-C₂₁-alkyl-C(O)- , preferentially C₃-C₂₁-alkyl- C(O) - ,
however, with the proviso that one of the radicals R⁶, R⁷ and R⁸ represents a radical CH₃- CH(OR⁴) - CH₂ - C(O) - .

12. The mixture according to any of claims 5 to 11,
wherein in the general formula (III) the radicals R⁶, R⁷ and R⁸, identical or different, each independently of one another represent
• a radical C₁₂-C₂₉-alkyl- C(O) - , especially C₁₉-C₂₉-alkyl- C(O) - ,
• a radical CH₃-CH(OR⁴)-CH₂-C(O)- , wherein the radical R⁴ has the meaning defined hereinabove, especially wherein the radical R⁴ represents hydrogen or a radical C₁-C₃₀-alkyl-C(O)- , especially C₁-C₂₁-alkyl-C(O)- , preferentially C₃-C₂₁-alkyl-C(O)- ,
however, with the proviso that one of the radicals R⁶, R⁷ and R⁸ represents a radical CH₃-CH(OR⁴)-CH₂-C(O)- .

13. The mixture according to any of claims 5 to 12,
wherein in the general formula (III) the radicals R⁶, R⁷ and R⁸, identical or different, each independently of one another represent
• a radical C₁-C₁₁-alkyl-C(O)- , especially C₂-C₁₁-alkyl-C(O)- ,
• a radical C₁₂-C₂₉-alkyl-C(O)- , especially C₁₉-C₂₉-alkyl-C(O)- ,
• a radical CH₃-CH(OR⁴)-CH₂-C(O)- , wherein the radical R⁴ has the meaning defined hereinabove, especially wherein the radical R⁴ represents hydrogen or a radical C₁-C₃₀-alkyl-C(O)- , especially C₁-C₂₁-alkyl-C(O)- , preferentially C₃-C₂₁-alkyl-C(O)- ,
however, with the proviso that one of the radicals R⁶, R⁷ and R⁸ represents a radical CH₃-CH(OR⁴)-CH₂-C(O)- and that one of the radicals R⁶, R⁷ and R⁸ represents a radical C₁-C₁₁-alkyl-C(O)- , especially C₂-C₁₁-alkyl-C(O)- , and that one of the radicals R⁶, R⁷ and R⁸ represents a radical C₁₂-C₂₉-alkyl-C(O) - , especially C₁₉-C₂₉-alkyl-C(O)- .

14. A mixture comprising at least three different lipids comprising structural units based on glycerides of 3-hydroxybutyric and/or 3-alkoxybutyric acids of the general formula (III), as defined in any of the proceeding claims.

15. A pharmaceutical composition, especially a drug or medicament, comprising a mixture according to any of claims 5 to 14.

16. The pharmaceutical composition according to claim 15 for the prophylactic and/or therapeutic treatment or for use in the prophylactic and/or therapeutic treatment of diseases of the human or animal body, especially diseases associated with a disorder of the energy metabolism, especially keto-body metabolism, such as especially craniocerebral trauma, stroke, hypoxia, cardiovascular diseases such as myocardial infarction, refeeding syndrome, anorexia, epilepsy, neurodegenerative diseases such as dementia, Alzheimer's disease, Parkinson's disease, multiple sclerosis and amyotrophic lateral sclerosis, fat metabolic diseases such as glucose transporter defect (GLUT1 defect), VL-FAOD and mitochondriopathies such as mitochondrial thiolase defect, Huntington's disease, cancers such as T-cell lymphomas, astrocytomas and glioblastomas, HIV, rheumatic diseases such as rheumatoid arthritis and arthritis urica, diseases of the gastrointestinal tract such as chronic inflammatory bowel diseases, especially ulcerative colitis and Crohn's disease, lyosomal storage diseases such as sphingolipidosis, especially Niemann-Pick disease, diabetes mellitus and effects or side-effects of chemotherapy.

17. A mixture according to any of Claims 5 to 14 for use in the prophylactic and/or therapeutic treatment of diseases of the human or animal body, especially diseases associated with a disorder of the energy metabolism, especially keto-body metabolism, such as especially craniocerebral trauma, stroke, hypoxia, cardiovascular diseases such as myocardial infarction, refeeding syndrome, anorexia, epilepsy, neurodegenerative diseases such as dementia, Alzheimer's disease, Parkinson's disease, multiple sclerosis and amyotrophic lateral sclerosis, fat metabolic diseases such as glucose transporter defect (GLUT1 defect), VL-FAOD and mitochondriopathies such as mitochondrial thiolase defect, Huntington's disease, cancers such as T-cell lymphomas, astrocytomas and glioblastomas, HIV, rheumatic diseases such as rheumatoid arthritis and arthritis urica, diseases of the gastrointestinal tract such as chronic inflammatory bowel diseases, especially ulcerative colitis and Crohn's disease, lyosomal storage diseases such as sphingolipidosis, especially Niemann-Pick disease, diabetes mellitus and effects or side-effects of chemotherapy.

18. Use of a mixture according to any of claims 5 to 14 for producing a medicament for the prophylactic and/or therapeutic treatment of diseases of the human or animal body, especially diseases associated with a disorder of the energy metabolism, especially keto-body metabolism, such as especially craniocerebral trauma, stroke, hypoxia, cardiovascular diseases such as myocardial infarction, refeeding syndrome, anorexia, epilepsy, neurodegenerative diseases such as dementia, Alzheimer's disease, Parkinson's disease, multiple sclerosis and amyotrophic lateral sclerosis, fat metabolic diseases such as glucose transporter defect (GLUT1 defect), VL-FAOD and mitochondriopathies such as mitochondrial thiolase defect, Huntington's disease, cancers such as T-cell lymphomas, astrocytomas and glioblastomas, HIV, rheumatic diseases such as rheumatoid arthritis and arthritis urica, diseases of the gastrointestinal tract such as chronic inflammatory bowel diseases, especially ulcerative colitis and Crohn's disease, lyosomal storage diseases such as sphingolipidosis, especially Niemann-Pick disease, diabetes mellitus and effects or side-effects of chemotherapy.

19. A food and/or a food product comprising a mixture according to any of claims 5 to 14.

20. Use of a mixture according to any of claims 5 to 14 in a food and/or a food product.

## Revendications

1. Procédé de préparation de lipides contenant des unités structurales à base de glycérides d'acides 3-hydroxy- et/ou 3-alcoxybutyriques,
où l'on fait réagir au moins un glycéride de formule générale (I)
R¹O-CH₂-CH(OR²)-CH₂-OR³ (I)
où, dans la formule générale (I), les radicaux R¹, R² et R³, identiques ou différents, représente, indépendamment les uns des autres,
• l'hydrogène,
• un radical C₁-C₁₁-alkyle-C(O)- , en particulier C₂-C₁₁-alkyle-C(O)- ,
• un radical C₁₂-C₂₉ -alkyle-C(O)- , en particulier C₁₉-C₂₉-alkyle-C(O)- ,
à condition toutefois qu'au moins un, de préférence au moins deux, des radicaux R¹, R² et R³ ne représentent pas un atome d'hydrogène,
avec au moins un dérivé d'acide 3-hydroxy- et/ou 3-alcoxybutyrique -de formule générale (II)
CH₃-CH(OR⁴)-CH₂-C(O)OR⁵ (II)
où, dans la formule générale (II),
• le radical R⁴ représente l'hydrogène ou un radical C₁-C₃₀-alkyle-C(O)- , en particulier C₁-C₂₁-alkyle-C(O)- , de préférence C₃-C₂₁-alkyle-C(O)- ,
• le radical R⁵ représente un hydrogène ou un C₁-C₄-alkyle, en particulier un C₁-C₄-alkyle, de préférence un méthyle ou un éthyle, de manière particulièrement préférée un éthyle,
la réaction étant effectuée en l'absence de solvants et/ou sans aucun solvant, et
la réaction étant effectuée en présence d'une enzyme servant de catalyseur,
de manière à obtenir, comme produit de réaction, un mélange d'au moins deux lipides différents l'un de l'autre et contenant des unités structurelles à base de glycérides d'acides 3-hydroxy et/ou 3-alcoxybutyriques de formule générale (III)
R⁶O-CH₂-CH(OR⁷)-CH₂-OR⁸ (III)
où, dans la formule générale (III), les radicaux R⁶ , R⁷ et R⁸, identiques ou différents, représente, indépendamment les uns des autres,
• un radical C₁-C₁₁-alkyle-C(O)- , en particulier C₂-C₁₁-alkyle-C(O)- ,
• un radical C₁₂-C₂₉-alkyle-C(O)- , en particulier C₁₉-C₂₉-alkyle-C(O)- ,
• un radical CH₃-CH(OR⁴)-CH₂-C(O)- , où le radical R⁴ a la signification indiquée précédemment,
à condition toutefois qu'un ou deux des radicaux R⁶, R⁷ et R⁸ représentent un radical CH₃-CH(OR⁴)-CH₂-C(O)- .

2. Procédé selon la revendication 1,
où dans la formule générale (II)
• le radical R⁴ représente un atome d'hydrogène ou un radical du type C₁-C₃₀-alkyle-C(O)- linéaire (à chaîne droite) ou ramifié, saturé ou mono- ou polyinsaturé, en particulier C₁-C₂₁-alkyle-C(O)- linéaire (à chaîne droite) ou ramifié, saturé ou mono- ou polyinsaturé, de préférence C₃-C₂₁-alkyle-C(O)- linéaire (à chaîne droite) ou ramifié, saturé ou mono- ou polyinsaturé,
• le radical R⁵ représente un C₁-C₄-alkyle, en particulier un C₁-C₄-alkyle, de préférence méthyle ou éthyle, de manière particulièrement préférée éthyle; et/ou
où dans la formule générale (II)
• le radical R⁴ représente un atome d'hydrogène ou un radical du type C₁-C₃₀-alkyle-C(O)- linéaire (à chaîne droite) ou ramifié, saturé ou mono- ou polyinsaturé, en particulier linéaire (à chaîne droite) ou ramifié, C₁-C₂₁-alkyle-C(O)- , de préférence C₃-C₂₁-alkyle-C(O)- , linéaire (à chaîne droite) ou ramifié, saturé ou mono- ou polyinsaturé,
• le radical R⁵ représente un groupe éthyle; et/ou
où, dans la formule générale (II), le radical R⁴ représente l'hydrogène et le radical R⁵ représente l'éthyle; et/ou
où l'on utilise, comme dérivé de l'acide 3-hydroxy et/ou 3-alcoxybutyrique de formule générale (II), l'ester éthylique de l'acide 3-hydroxybutyrique (3-hydroxybutyrate d'éthyle) de formule CH₃-CH(OH)-CH₂-C(O)OC₂H₅ ; et/ou
le dérivé d'acide 3-hydroxy et/ou 3-alcoxybutyrique de formule générale (II) étant utilisé -sous forme racémique ou sous forme d'énantiomère- (R); en particulier la configuration (R) étant relative à l'atome de carbone en position-3 et/ou en particulier la configuration (R) étant relative à l'atome de carbone portant le radical OR⁴.

3. Procédé selon l'une quelconque des revendications précédentes,
où le catalyseur est recyclé après réaction; et/ou
où l'enzyme est choisie parmi les synthétases (ligases), les catalases, les estérases, les lipases et leurs combinaisons; et/ou
où l'enzyme est dérivée de *Candida antarctica, Mucor miehei* (*Rhizomucor miehei*)*, Thermomyces lanuginosus, Candida rugosa, Aspergillus oryzae, Pseudomonas cepacia, Pseudomonas fluorescens, Rhizopus delemar* et *Pseudomonas* sp. ainsi que leurs combinaisons, de préférence *Candida antarctica, Mucor miehei* (*Rhizomucor miehei*) et *Thermomyces lanuginosus;* et/ou
où l'enzyme est utilisée sous forme immobilisée, en particulier immobilisée sur un support, de préférence sur un support polymère, de préférence sur un support organique polymère, de manière particulièrement préférée avec des propriétés hydrophobes, de manière tout particulièrement préférée sur un support à base de résine poly(méth)acrylique; et/ou
où l'enzyme est recyclée après la réaction; et/ou
où réaction est effectuée en présence d'une enzyme comme catalyseur à des températures dans la plage de 10 °C à 80 °C, en particulier dans la plage de 20 °C à 80 °C, de préférence dans la plage de 25 °C à 75 °C, de manière particulièrement préférée dans la plage de 45 °C à 75 °C, de manière tout particulièrement préférée dans la plage de 50 °C à 70 °C; et/ou
où l'enzyme est utilisée en des quantités, par rapport à la quantité totale des composés de départ (I) et (II), dans la plage de 0,001 % en poids à 20 % en poids, en particulier dans la plage de 0,01 % en poids à 15 % en poids, de préférence dans la plage de 0,1 % en poids à 15 % en poids-, de préférence dans la plage de 0,5 % en poids à 10 % en poids; et/ou
où la réaction est effectuée en présence d'une enzyme comme catalyseur à une pression dans la plage de 0,0001 bar à 10 bars, en particulier dans la plage de 0,001 bar à 5 bars, de préférence dans la plage de 0,01 bar à 2 bars, de manière particulièrement préférée dans la plage de 0,05 bar à 1 bar, tout particulièrement à environ 1 bar.

4. Procédé selon l'une quelconque des revendications précédentes,
où, dans le cas où dans la formule générale (II) le radical R⁵ désigne l'hydrogène à la place de l'acide libre, on utilise son anhydride de formule générale (IIa)
[CH₃-CH(OR⁴)-CH₂-C(O)]₂O (IIa)
où, dans la formule générale (IIa), le radical R⁴ a la signification indiquée précédemment.

5. Mélange comprenant au moins deux lipides différents l'un de l'autre et contenant des unités structurelles à base de glycérides d'acides 3-hydroxy et/ou 3-alcoxybutyriques de formule générale (III)
R⁶O- CH₂ - CH(OR⁷) - CH₂ - OR⁸ (III)
où, dans la formule générale (III), les radicaux R⁶, R⁷ et R⁸, identiques ou différents, représente, indépendamment les uns des autres,
• un radical C₁-C₁₁-alkyle-C(O)- , en particulier C₂-C₁₁-alkyle-C(O)- ,
• un radical C₁₂-C₂₉-alkyle-C(O - , en particulier C₁₉-C₂₉-alkyle-C(O)- ,
• un radical CH₃-CH(OR⁴)-CH₂-C(O)- , où le radical R⁴ a la signification indiquée précédemment, en particulier où le radical R⁴ représente l'hydrogène ou un radical C₁-C₃₀-alkyle-C(O)- , en particulier C₁-C₂₁-alkyle-C(O)- , de préférence C₃-C₂₁-alkyle-C(O)- ,
à condition toutefois que l'un ou deux des radicaux R⁶, R⁷ et R⁸ représentent un radical CH₃-CH(OR⁴)-CH₂-C(O)- .

6. Mélange selon la revendication 5,
où, dans la formule générale (III), les radicaux R⁶, R⁷ et R⁸, identiques ou différents, représente, indépendamment les uns des autres,
• un radical du type C₁-C₁₁-alkyle-C(O)- linéaire (chaîne droite) ou ramifié, saturé ou mono- ou polyinsaturé , notamment C₂-C₁₁-alkyle-C(O)- linéaire (chaîne droite) ou ramifié, saturé ou mono- ou polyinsaturé ,
• un radical du type C₁₂-C₂₉-alkyle-C(O)- linéaire (chaîne droite) ou ramifié, saturé ou mono- ou polyinsaturé , notamment C₁₉-C₂₉-alkyle-C(O)- linéaire (chaîne droite) ou ramifié, saturé ou mono- ou polyinsaturé ,
• un radical du type linéaire (chaîne droite) ou ramifié, saturé ou mono- ou polyinsaturé CH₃-CH(OR⁴)-CH₂-C(O)-, où le radical R⁴ a la signification donnée précédemment, en particulier où le radical R⁴ représente l'hydrogène ou un radical C₁-C₃₀-alkyle-C(O)- , en particulier C₁-C₂₁-alkyle-C(O)- , de préférence C₃-C₂₁-alkyle-C(O)- ,
à condition toutefois qu'au moins l'un des radicaux R⁶ , R⁷ et R⁸ représente un radical CH₃-CH(OR⁴)-CH₂-C(O)- .

7. Mélange selon la revendication 5 ou 6,
où, dans la formule générale (III), les radicaux R⁶, R⁷ et R⁸, identiques ou différents, représente, indépendamment les uns des autres,
• un radical C₁-C₁₁-alkyle-C(O)- , en particulier C₂-C₁₁-alkyle-C(O)- ,
• un radical C₁₂-C₂₉-alkyle-C(O)- , en particulier C₁₉-C₂₉-alkyle-C(O)- ,
• un radical CH₃-CH(OR⁴)-CH₂-C(O)- , où le radical R⁴ a la signification indiquée précédemment, en particulier où le radical R⁴ représente l'hydrogène ou un radical C₁-C₃₀-alkyle-C(O)- , en particulier C₁-C₂₁-alkyle-C(O)- , de préférence C₃-C₂₁-alkyle-C(O)- ,
à condition toutefois que deux radicaux R⁶, R⁷ et R⁸ représentent un radical CH₃-CH(OR⁴)-CH₂-C(O)-.

8. Mélange selon l'une quelconque des revendications 5 à 7,
où, dans la formule générale (III), les radicaux R⁶, R⁷ et R⁸, identiques ou différents, représente, indépendamment les uns des autres,
• un radical C₁-C₁₁-alkyle-C(O)- , en particulier C₂-C₁₁-alkyle-C(O)- ,
• un radical CH₃-CH(OR⁴)-CH₂-C(O)- , où le radical R⁴ a la signification indiquée précédemment, en particulier où le radical R⁴ représente l'hydrogène ou un radical C₁-C₃₀-alkyle-C(O)- , en particulier C₁-C₂₁-alkyle-C(O)- , de préférence C₃-C₂₁-alkyle-C(O)- ,
à condition toutefois que deux radicaux R⁶, R⁷ et R⁸ représentent un radical CH₃-CH(OR⁴)-CH₂-C(O)-.

9. Mélange selon l'une quelconque des revendications 5 à 8,
où, dans la formule générale (III), les radicaux R⁶, R⁷ et R⁸, identiques ou différents, représente, indépendamment les uns des autres,
• un radical C₁₂-C₂₉-alkyle-C(O)- , en particulier C₁₉-C₂₉-alkyle-C(O)- ,
• un radical CH₃-CH(OR⁴)-CH₂-C(O)- , où le radical R⁴ a la signification indiquée précédemment, en particulier où le radical R⁴ représente l'hydrogène ou un radical C₁-C₃₀-alkyle-C(O)- , en particulier C₁-C₂₁-alkyle-C(O)- , de préférence C₃-C₂₁-alkyle-C(O)- ,
à condition toutefois que deux radicaux R⁶, R⁷ et R⁸ représentent un radical CH₃-CH(OR⁴)-CH₂-C(O)- .

10. Mélange selon l'une quelconque des revendications 5 à 9,
où, dans la formule générale (III), les radicaux R⁶, R⁷ et R⁸, identiques ou différents, représente, indépendamment les uns des autres,
• un radical C₁-C₁₁-alkyle-C(O)- , en particulier C₂-C₁₁-alkyle-C(O)- ,
• un radical C₁₂-C₂₉-alkyle-C(O)- , en particulier C₁₉-C₂₉-alkyle-C(O)- ,
• un radical CH₃-CH(OR⁴)-CH₂-C(O)- , où le radical R⁴ a la signification indiquée précédemment, en particulier où le radical R⁴ représente l'hydrogène ou un radical C₁-C₃₀-alkyle-C(O)- , en particulier C₁-C₂₁-alkyle-C(O)- , de préférence C₃-C₂₁-alkyle-C(O)- ,
à condition toutefois que l'un des radicaux R⁶, R⁷ et R⁸ représente un radical CH₃-CH(OR⁴)-CH₂-C(O)- .

11. Mélange selon l'une quelconque des revendications 5 à 10,
où, dans la formule générale (III), les radicaux R⁶, R⁷ et R⁸, identiques ou différents, représente, indépendamment les uns des autres,
• un radical C₁-C₁₁-alkyle-C(O)- , en particulier C₂-C₁₁-alkyle-C(O)- ,
• un radical CH₃-CH(OR⁴)-CH₂-C(O)- , où le radical R⁴ a la signification indiquée précédemment, en particulier où le radical R⁴ représente l'hydrogène ou un radical C₁-C₃₀-alkyle-C(O)- , en particulier C₁-C₂₁-alkyle-C(O)- , de préférence C₃-C₂₁-alkyle-C(O)- ,
à condition toutefois que l'un des radicaux R⁶, R⁷ et R⁸ représente un radical CH₃-CH(OR⁴)-CH₂-C(O)-.

12. Mélange selon l'une quelconque des revendications 5 à 11,
où, dans la formule générale (III), les radicaux R⁶, R⁷ et R⁸, identiques ou différents, représente, indépendamment les uns des autres,
• un radical C₁₂-C₂₉-alkyle-C(O)- , en particulier C₁₉-C₂₉-alkyle-C(O)- ,
• un radical CH₃-CH(OR⁴)-CH₂-C(O)- , où le radical R⁴ a la signification indiquée précédemment, en particulier où le radical R⁴ représente l'hydrogène ou un radical C₁-C₃₀-alkyle-C(O)- , en particulier C₁-C₂₁-alkyle-C(O)- , de préférence C₃-C₂₁-alkyle-C(O)- ,
à condition toutefois que l'un des radicaux R⁶, R⁷ et R⁸ représente un radical CH₃-CH(OR⁴)-CH₂-C(O)- .

13. Mélange selon l'une quelconque des revendications 5 à 12,
où, dans la formule générale (III), les radicaux R⁶, R⁷ et R⁸, identiques ou différents, représente, indépendamment les uns des autres,
• un radical C₁-C₁₁-alkyle - C(O) - , en particulier C₂-C₁₁-alkyle - C(O) - ,
• un radical C₁₂-C₂₉-alkyle - C(O) - , en particulier C₁₉-C₂₉-alkyle - C(O) - ,
• un radical CH₃-CH(OR⁴)-CH₂-C(O)- , où le radical R⁴ a la signification indiquée précédemment, en particulier où le radical R⁴ représente l'hydrogène ou un radical C₁-C₃₀-alkyle - C(O) - , en particulier C₁-C₂₁-alkyle - C(O) - , de préférence C₃-C₂₁-alkyle - C(O) - ,
à condition toutefois que l'un des radicaux R⁶, R⁷ et R⁸ représente un radical CH₃- CH(OR⁴) - CH₂ - C(O) - et que l'un des radicaux R⁶, R⁷ et R⁸ représente un radical C₁-C₁₁-alkyle - C(O) - , en particulier C₂-C₁₁-alkyle - C(O) - , et que l'un des radicaux R⁶, R⁷ et R⁸ représente un radical C₁₂-C₂₉-alkyle - C(O) -, en particulier C₁₉-C₂₉-alkyle - C(O) - .

14. Mélange comprenant au moins trois lipides différents les uns des autres et contenant des unités structurelles à base de glycérides d'acides 3-hydroxy et/ou 3-alcoxybutyriques de formule générale (III), comme défini dans les revendications précédentes .

15. Composition pharmaceutique, en particulier médicament ou médicament, comprenant un mélange selon l'une quelconque des revendications 5 à 14.

16. Composition pharmaceutique selon la revendication 15 pour le traitement prophylactique et/ou thérapeutique, respectivement pour l'utilisation dans le traitement de l'organisme. pour son utilisation dans le traitement prophylactique et/ou thérapeutique de maladies du corps humain ou animal, en particulier de maladies liées à un trouble du métabolisme énergétique, en particulier du métabolisme des corps cétoniques, comme en particulier les traumatismes crâniens, les accidents vasculaires cérébraux, les hypoxies, les maladies cardiovasculaires comme l'infarctus du myocarde, le syndrome de réalimentation, les anorexies, l'épilepsie, les maladies neurodégénératives comme la démence, la maladie d'Alzheimer, la maladie de Parkinson, la sclérose en plaques et la sclérose latérale amyotrophique, les maladies du métabolisme lipidique telles que le déficit en transporteur de glucose (déficit GLUT1), la VL-FAOD et les mitochondriopathies telles que le déficit en thiolase mitochondriale, la chorée de Huntington, les cancers tels que les lymphomes à cellules T, les astrocytomes et les glioblastomes, le VIH, les maladies rhumatismales telles que la polyarthrite rhumatoïde et l'arthrite urique, les maladies du tractus gastro-intestinal telles que les maladies inflammatoires chroniques de l'intestin, notamment la colite ulcéreuse et la maladie de Crohn, les maladies du stockage lyosomique telles que les sphingolipidoses, notamment la maladie de Niemann-Pick, le diabète sucré et les effets ou effets secondaires des chimiothérapies.

17. Mélange selon l'une des revendications 5 à 14 pour son utilisation dans le traitement prophylactique et/ou thérapeutique de maladies du corps humain ou animal, notamment de maladies liées à un trouble du métabolisme énergétique, en particulier du métabolisme des corps cétoniques, comme notamment les traumatismes crâniens, les accidents vasculaires cérébraux, les hypoxies, les maladies cardiovasculaires comme l'infarctus du myocarde, le syndrome de réalimentation, les anorexies, l'épilepsie, les maladies neurodégénératives comme la démence, la maladie d'Alzheimer, la maladie de Parkinson, la sclérose en plaques et la sclérose latérale amyotrophique, les maladies du métabolisme lipidique telles que le déficit en transporteur de glucose (déficit GLUT1), la VL-FAOD et les mitochondriopathies telles que le déficit en thiolase mitochondriale, la chorée de Huntington, les cancers tels que les lymphomes à cellules T, les astrocytomes et les glioblastomes, le VIH, les maladies rhumatismales telles que la polyarthrite rhumatoïde et l'arthrite urique, les maladies du tractus gastro-intestinal telles que les maladies inflammatoires chroniques de l'intestin, notamment la colite ulcéreuse et la maladie de Crohn, les maladies du stockage lyosomique telles que les sphingolipidoses, notamment la maladie de Niemann-Pick, le diabète sucré et les effets ou effets secondaires des chimiothérapies.

18. Utilisation d'un mélange selon l'une des revendications 5 à 14 pour la fabrication d'un médicament destiné au traitement prophylactique et/ou thérapeutique de maladies du corps humain ou animal, en particulier de maladies liées à un trouble du métabolisme énergétique, en particulier le métabolisme des corps cétoniques, comme en particulier les traumatismes crâniens, les accidents vasculaires cérébraux, les hypoxies, les maladies cardiovasculaires comme l'infarctus du myocarde, le syndrome de réalimentation, les anorexies, l'épilepsie, les maladies neurodégénératives comme la démence, la maladie d'Alzheimer, la maladie de Parkinson, la sclérose en plaques et la sclérose latérale amyotrophique, les maladies du métabolisme lipidique telles que le défaut du transporteur de glucose (défaut GLUT1), VL-FAOD et les mitochondriopathies telles que le défaut de thiolase mitochondriale, la chorée de Huntington, les cancers tels que les lymphomes à cellules T, les astrocytomes et les glioblastomes, le VIH, les maladies rhumatismales telles que la polyarthrite rhumatoïde et l'arthrite urique, les maladies du tractus gastro-intestinal telles que les maladies inflammatoires chroniques de l'intestin, notamment la colite ulcéreuse et la maladie de Crohn, les maladies du stockage lyosomique telles que les sphingolipidoses, notamment la maladie de Niemann-Pick, le diabète sucré et les effets ou effets secondaires des chimiothérapies.

19. Produit alimentaire et/ou nutritionnel comprenant un mélange selon l'une quelconque des revendications 5 à 14.

20. Utilisation d'un mélange selon l'une quelconque des revendications 5 à 14 dans un produit alimentaire et/ou nutritionnel.
